(19)

Europäisches
Patentamt
European
Patent Office
Office européen
des brevets

(11) **EP 4 349 459 A1**

(12) **EUROPEAN PATENT APPLICATION**

(43) Date of publication:
**10.04.2024 Bulletin 2024/15**

(21) Application number: **22199848.7**

(22) Date of filing: **05.10.2022**

(51) International Patent Classification (IPC):
***B01D 61/00*** (2006.01) ***B01D 61/18*** (2006.01)
***B01D 69/02*** (2006.01)

(52) Cooperative Patent Classification (CPC):
**B01D 61/005; B01D 61/0022; B01D 61/18;**
**B01D 69/02;** B01D 2311/06; B01D 2311/12;
B01D 2311/14; B01D 2311/243; B01D 2311/25;
B01D 2325/20

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **BioSpring Gesellschaft für**
**Biotechnologie mbH**
**60386 Frankfurt am Main (DE)**

(72) Inventor: **AYGÜN, Hüseyn**
**60386 Frankfurt am Main (DE)**

(74) Representative: **Kraus & Lederer PartGmbB**
**Thomas-Wimmer-Ring 15**
**80539 München (DE)**

(54) **ULTRAFILTRATION METHODS USING OSMOLYTES ON THE PERMEATE SIDE**

(57) The present invention relates to methods for ultrafiltration of molecules of interest and to related uses, respectively, which advantageously employ osmolytes. Among others, the invention provides methods and uses for concentrating molecules of interest via ultrafiltration.

EP 4 349 459 A1

**Description**

FIELD OF THE INVENTION

The present invention relates to the field of ultrafiltration of molecules of interest. The methods employ osmotically active (non-permeating) molecules ("osmolytes") on the permeate side of an ultrafiltration membrane. Among others, this advantageously enhances flux through the ultrafiltration membrane and e.g. allows an improved concentrating of solutions comprising molecules of interest.

BACKGROUND

Ultrafiltration is an important technical method that involves the use of a semipermeable membrane. Similarly as in related separation techniques such as microfiltration, said membrane allows to retain the (macro)molecules of interest in the retentate, whereas low molecular weight molecules (like salt, organic solvents or other low molecular weight by-products) as well as water molecules that pass through the ultrafiltration membrane are found in the permeate.

Accordingly, there are in principle two sites of main interest: The retentate side of the ultrafiltration membrane as well as the permeate side of the ultrafiltration membrane. Moreover, according to the respective goal to be achieved, either the permeate (e.g. in the clearing of water) or the retentate (e.g. in the concentration of molecules of interest) is the fraction of interest.

Generally, ultrafiltration is widely used with macromolecular molecules, having a molecular weight of from about 1 kDa to about 1000 kDa. A person of skill in the art will readily appreciate that ultrafiltration is of particular interest e.g. for the purification and/or concentration of molecules of interest, in particular molecules of interest that qualify as active pharmaceutical ingredient (API), and thus especially also in the preparation of drug products / medicaments.

Other medical applications using ultrafiltration include blood dialysis - and industrial applications of ultrafiltration e.g. include food / beverage processing as well as waste water treatment.

Particular applications of ultrafiltration include the transfer of molecules of interest into different buffers/solutions (e.g. by continuously applying a flow of a new buffer thus diluting out the previous buffer through the ultrafiltration membrane) - others the separation of molecules of interest from (undesired) molecules of low molecular weight as those pass through the ultrafiltration membrane (such as in a desalting process).

Generally, there are two main modes for carrying out ultrafiltration, namely direct filtration and tangential filtration, the latter also being called tangential flow filtration.

In particular, "diafiltration", e.g. as a particular mode of ultrafiltration, is a process that includes the removal of permeable components such as salts, organic solvents or other undesired molecules from a solution - and ultrafiltration/diafiltration ("UF/DF") is a common variant of ultrafiltration. Diafiltration involves a so-called diluent or replacement buffer, which is (continuously) combined with the retentate and which is (re-)introduced into the ultrafiltration process.

Further important applications of ultrafiltration include the concentrating of macromolecules such as molecules of interest, which may e.g. be necessary and/or desirable during purification of said molecules of interest and particularly also in the process of preparing drug products / medicaments.

Important exemplary molecules of interest that are widely used in ultrafiltration methods include polypeptides / proteins, plasmids, mRNA as well as oligonucleotides.

Notably, in the preparation of drug products e.g. therapeutic oligonucleotides are commonly provided as lyophilized solid material and are later re-dissolved for final aqueous formulation. Historically, one reason for employing such a lyophilization step may have been that a lyophilisate was often considered to be of a higher stability than an aqueous oligonucleotide solution, whereas nowadays also aqueous solutions are generally considered sufficiently stable as well (see Muslehiddinoglu et al.). In any case, prior to lyophilization the oligonucleotide solution is typically desalted and concentrated via ultrafiltration / diafiltration (UF/DF).

One particular reason to provide APIs such as oligonucleotide APIs as solids is the fact that ultrafiltration often is not able to deliver the oligonucleotide in a suitable concentration (e.g. > 150mg/ml) for final drug product formulation. Furthermore, the final concentration will also influence the lyophilization capacity. This can be the bottleneck of production for batches having a very large volume.

Therefore, a high final oligonucleotide concentration after ultrafiltration or UF/DF may generally be desirable - such that there is generally a need in the art for methods allowing the provision of solutions comprising molecules of interest in high concentrations.

According to the literature, for a typical antisense oligonucleotide, a final concentration of 40 - 100 mg/mL can be achieved with standard UF/DF processes depending on the sequence and composition of the oligonucleotide (see Muslehiddinoglu et al.), whereas experimental data obtained in the context with this application indicate that typically a maximal concentration of around 60 - 90 mg/mL can be achieved for different oligonucleotides.

At any rate, as already indicated above, such concentrations are regularly not high enough for the preparation of the resulting liquid formulations as medicaments, which is why lyophilization is regularly nevertheless required. Consequently, there is a general need in the art for providing improved ultrafiltration methods that overcome said limitations by e.g. facilitating the production of highly concentrated molecule of interest solutions by ultrafiltration methods only and without the need of employing ultrafiltration.

In further detail as to the technique of ultrafiltration, important parameters to describe an ultrafiltration procedure are feed pressure, retentate pressure, permeate pressure, the differential pressure and the transmembrane pressure. All these parameters can be recorded continuously by the system (if there is a need). The feed pressure is mainly determined by the feed flow and the viscosity of the pumped solution while retentate and permeate pressure can be controlled via tunable valves. The differential pressure $\Delta p$ is defined in the following way:

$$\Delta p = \text{Feed pressure} - \text{retentate pressure}$$

The transmembrane pressure ("TMP") which is the driving force for material transfer through the membrane may be calculated by the following equation:

$$\text{TMP} = (\text{Feed pressure} + \text{retentate pressure})/2 - \text{Permeate pressure}$$

Since the sum of the retentate flow rate (recirculation flow rate) and the permeate flow rate (flux) equals the feed flow rate, the flux through the membrane can be controlled either by increasing the feed flow rate or by decreasing the retentate flow rate (increasing retentate pressure).

During ultrafiltration, typical process control modes are either the TMP control mode or the flux control mode.

During ultrafiltration, the viscosity of the pumped solution will generally rise as well as the apparent concentration of the product on the membrane surface. If feed flow rate is kept constant and retentate valves are not opened for compensation of the increasing viscosity this will lead to an increased feed pressure and the process will stop due to the feed pressure limit and/or fouling. However, if the feed flow rate is automatically lowered or retentate valves opened the flux through the membrane will be lowered up to a point where no more flux through the membrane can be observed.

This further describes why concentrating an oligonucleotide solution above a certain limit may not be possible with classical UF/DF approaches, and why there indeed is a need to improve ultrafiltration methods that overcome such limitations.

The present invention addresses all of the above objects and needs by providing respective solutions as defined in the claims.

SUMMARY OF THE INVENTION

Among others, the present invention provides new procedures that use principles of osmosis such as the osmotic flow generated by "osmotically active molecules", which are interchangeably referred to herein as "osmolytes".

In more detail, the present invention relates to methods for ultrafiltration of molecules of interest and to related uses, respectively, which advantageously employ osmolytes. Among others, the invention provides methods and uses for concentrating molecules of interest via ultrafiltration.

Non-limiting examples of said osmolytes include polymers, which are preferably selected from the group consisting of polyethylene glycol (PEG), polyacrylic acid (PAA), polyvinyl pyrrolidone, carboxymethyl cellulose, polyvinyl alcohol, or a mixture thereof, and which can be provided on the permeate side of the ultrafiltration membrane to overcome the above limitation.

The inventors of the present application found, among others, that it is possible to advantageously use principles of osmosis in the ultrafiltration of molecules of interest. In particular, osmolytes are provided on the permeate side of the ultrafiltration membrane to enhance flux through the ultrafiltration membrane. In doing so, it e.g. becomes possible to improve methods for concentrating molecules of interest using ultrafiltration and to achieve much higher concentrations of molecules of interest than with standard ultrafiltration techniques.

Generally, the present invention provides subject-matter as defined in the present claims.

Moreover, the invention particularly also relates to the subject matter defined in the following items [1] to [141]:

[1] A method for ultrafiltrating a solution comprising molecules of interest, the method comprising a step of contacting the permeate side of the ultrafiltration membrane with a solution comprising osmolytes having a molecular weight that is higher than the molecular weight cut-off (MWCO) of the ultrafiltration membrane, wherein the solution on the permeate side of the ultrafiltration membrane has a higher osmolarity than the solution on the retentate side of the ultrafiltration membrane.

[2] A method for ultrafiltrating a solution comprising molecules of interest, characterized in that it comprises providing a higher osmolarity on the permeate side of the ultrafiltration membrane than on the retentate side of the ultrafiltration membrane by providing osmolytes, which have a molecular weight that is higher than the MWCO of the ultrafiltration membrane, on the permeate side of the ultrafiltration membrane.

[3] A method for ultrafiltrating a solution comprising molecules of interest, characterized in that it comprises providing osmolytes, which have a molecular weight that is higher than the MWCO of the ultrafiltration membrane, on the permeate side of the ultrafiltration membrane in order to improve ultrafiltration.

[4] A method for ultrafiltrating a solution comprising molecules of interest, characterized in that it comprises generating an osmotic flow through the ultrafiltration membrane, wherein said osmotic flow is generated by osmolytes provided on the permeate side of the ultrafiltration membrane, which have a molecular weight that is higher than the MWCO of the ultrafiltration membrane.

[5] A method for enhancing ultrafiltration of a solution comprising molecules of interest, the method comprising a step of contacting the permeate side of the ultrafiltration membrane with a solution comprising osmolytes having a molecular weight that is higher than the MWCO of the ultrafiltration membrane to enhance flux through the ultrafiltration membrane.

[6] An ultrafiltration method for preparing a concentrated solution of molecules of interest from a starting solution of said molecules of interest,

wherein the method comprises a step of contacting the permeate side of the ultrafiltration membrane with a solution comprising osmolytes having a molecular weight that is higher than the MWCO of the ultrafiltration membrane,

particularly wherein the solution on the permeate side of the ultrafiltration membrane has a higher osmolarity than the solution on the retentate side of the ultrafiltration membrane.

[7] An ultrafiltration method for preparing a concentrated solution of molecules of interest from a starting solution of said molecules of interest, wherein the method is characterized in that concentrating comprises providing an osmolyte on the permeate side of the ultrafiltration membrane which osmolyte has a molecular weight that is higher than the MWCO of the ultrafiltration membrane.

[8] The method of any one of the preceding items, wherein the method is a method for concentrating a solution comprising molecules of interest via ultrafiltration, in particular wherein ultrafiltration is tangential flow filtration (TFF) or cross-flow filtration (CFF), respectively.

[9] The method of any one of the preceding items, wherein the method allows concentrating said molecules of interest to a concentration of at least 100 mg/ml, particularly to a concentration of at least 150 mg/ml.

[10] The method of any one of the preceding items, wherein the method allows concentrating said molecules of interest to a concentration of at least 200 mg/ml, particularly to a concentration of at least 250 mg/ml.

[11] The method of any one of the preceding items, wherein the method allows concentrating said molecules of interest to a concentration of at least 300 mg/ml, particularly to a concentration of at least 350 mg/ml.

[12] The method of any one of the preceding items, wherein the method allows concentrating said molecules of interest to a concentration of at least 400 mg/ml.

[13] The method of any one of the preceding items, wherein the method allows concentrating said molecules of interest to a concentration of up to 500 mg/ml for polynucleotides as the molecules of interest and/or of up to 1000 mg/ml for polypeptides as the molecules of interest.

[14] The method of any one of the preceding items, wherein said molecules of interest are i) optionally modified oligonucleotides, or ii) optionally modified polypeptides.

[15] The method of any one of the preceding items, wherein said molecules of interest are oligonucleotides, which may or may not be modified.

[16] The method of item [15], wherein said optionally modified oligonucleotide is selected from the group consisting of DNA, RNA, and mixtures thereof (such as heteroduplexes), particularly wherein said DNA and/or RNA may be single stranded or double stranded, especially wherein the RNA is selected from the group consisting of RNAzymes, siRNA, DsiRNA, shRNA, miRNA, anti-miRNA, mRNA, rRNA, tRNA and sgRNA

[17] The method of item [15] or [16], wherein said oligonucleotide is selected from the group consisting of optionally modified DNA, optionally modified RNA, optionally modified mixtures thereof (such as optionally modified heteroduplexes).

[18] The method of item [15] or [16], wherein said oligonucleotide is optionally modified ssDNA.

[19] The method of item [15] or [16], wherein said oligonucleotide is optionally modified dsDNA.

[20] The method of item [15] or [16], wherein said oligonucleotide is optionally modified ssRNA.

[21] The method of item [15] or [16], wherein said oligonucleotide is optionally modified dsRNA.

[22] The method of item [15] or [16], wherein said oligonucleotide is selected from the group consisting of optionally modified siRNA, optionally modified DsiRNA, optionally modified shRNA, optionally modified miRNA, optionally modified anti-miRNA, optionally modified mRNA, optionally modified rRNA, optionally modified tRNA and optionally modified sgRNA.

[23] The method of item [15] or [16], wherein said oligonucleotide is selected from the group consisting of an optionally modified plasmid, an optionally modified aptamer, an DNAzyme, an RNAzyme, and an optionally modified antisense oligonucleotide.

[24] The method of any one of items [15] to [23], wherein said optionally modified oligonucleotide has a length of from 10 to 6000 nucleotides, preferably 10 to 300 nucleotides, particularly of from 14 to 130 nucleotides, such as of from 16 to 110 nucleotides

[25] The method of any one of items [15] to [23], wherein said optionally modified oligonucleotide has a molecular weight of from 3 to 90 kDa, particularly of from 4 to 39 kDa, especially of from 5 to 33 kDa.

[26] The method of any one of items [15] to [25], wherein said oligonucleotides are a mixture of optionally modified oligonucleotides, such as a double stranded oligonucleotides which is a mixture of two single stranded oligonucleotides, or a mixture of various oligonucleotides.

[27] The method of any one of items [15] to [25], wherein said optionally modified oligonucleotides are a mixture of any of the individual types of optionally modified oligonucleotides defined in any of items [17] to [23].

[28] The method of any one of items [1] to [14], wherein said molecules of interest are polypeptides, which may or may not be modified.

[29] The method of item [28], wherein said molecule of interest is an optionally modified polypeptide, which is selected from the group consisting of an enzyme, a cell receptor ligand, a protein ligand, a signal transduction protein, a cytokine, an antibody, an antibody fragment, an antibody domain, a diabody, an scFv fragment, an sc(Fv)2 fragment, and a chimeric antibody.

[30] The method of any one of items [28] to [29], wherein said polypeptide is an optionally modified enzyme.

[31] The method of any one of items [28] to [29], wherein said polypeptide is an optionally modified cell receptor ligand

[32] The method of any one of items [28] to [29], wherein said polypeptide is an optionally modified cell protein ligand.

[33] The method of any one of items [28] to [29], wherein said polypeptide is an optionally modified signal transduction protein.

[34] The method of any one of items [28] to [29], wherein said polypeptide is an optionally modified cytokine.

[35] The method of any one of items [28] to [29], wherein said polypeptide is an optionally modified antibody.

[36] The method of any one of items [28] to [29], wherein said polypeptide is an optionally modified antibody fragment or an optionally modified antibody domain.

[37] The method of any one of items [28] to [29], wherein said polypeptide is an optionally modified scFv fragment or an optionally modified sc(Fv)2 fragment.

[38] The method of any one of items [28] to [29], wherein said polypeptide is an optionally modified chimeric antibody.

[39] The method of any one of items [28] to [38], wherein said polypeptide comprises from 90 to 1800 amino acid residues, particularly from 135 to 1800 amino acid residues, especially from 135 to 1350 amino acid residues.

[40] The method of any one of items [28] to [39], wherein said polypeptide has a molecular weight of from 10 to 200 kDa, particularly from 15 to 200 kDa, especially from 15 to 150 kDa.

[41] The method of any one of items [28] to [40], wherein said polypeptides are a mixture of polypeptides.

[42] The method of any one of items [28] to [41], wherein said polypeptides are a mixture of any of the individual types of polypeptides defined in any of items [30] to [38].

[43] The method of any one of items [1] to [42], wherein said molecule of interest has a molecular weight of from 10 to 200 kDa.

[44] The method of any one of items [1] to [43], wherein said molecule of interest is modified.

[45] The method of any one of items [1] to [27], wherein said molecule of interest is a modified oligonucleotide comprising one or more modifications selected from the group consisting of methylation, carboxymethylation, acetylation, methoxyaminomethylation, methoxycarbonylation, thiolation, adenylation, polyadenylation, base modifications, backbone modifications, sugarmodifications (in particular glycosylation), linkers, lipids, peptides, and dyes.

[46] The method of any one of items [1] to [27] and [45] wherein said one or more modifications are independently selected from base-modifications, backbone modifications, sugar modifications, and other modifications;

> i) optionally wherein said base modification is independently selected from the group consisting of hypoxanthine, inosine, 8-oxo-adenine, 7-substituted derivatives thereof, dihydrouracil, pseudouracil, 2-thiouracil, 4-thiouracil, 5-aminouracil, 5-(C1-C6)-alkyluracil, 5-methyluracil, 5-(C2-C6)-alkenyluracil, 5-(C2-C6)-alkynyluracil, 5-(hydroxymethyl)uracil, 5-chlorouracil, 5-fluorouracil, 5-bromouracil, 5-hydroxycytosine, 5-(C1-C6)-alkylcytosine, 5-methylcytosine, 5-(C2-C6)-alkenylcytosine, 5-(C2-C6)-alkynylcytosine, 5-chlorocytosine, 5-fluorocytosine, 5-bromocytosine, N2-dimethylguanine, 7-deazaguanine, 8-azaguanine, 7-deaza-7-substituted guanine, 7-deaza-7-(C2-C6)alkynylguanine, 7-deaza-8-substituted guanine, 8-hydroxyguanine, 6-thioguanine, 8-oxoguanine, 2-aminopurine, 2-amino-6-chloropurine, 2,4-diaminopurine, 2,6-diaminopurine, 8-azapurine, substituted 7-deazapurine, 7-deaza-7-substituted purine, 7-deaza-8-substituted purine, hydrogen (abasic residue), and any combination thereof;
> and/or

> ii) optionally wherein said backbone modification is preferably selected from the group consisting of modified versions of the phosphodiester present in DNA or RNA, preferably selected from phosphorothioate (PS), phosphorodithioate (PS2), phosphonoacetate (PACE), phosphonoacetamide (PACA), thiophosphonoacetate, thiophosphonoacetamide, phosphorothioate prodrug, H-phosphonate, methyl phosphonate, methyl phosphonothioate, methyl phosphate, methyl phosphorothioate, ethyl phosphate, ethyl phosphorothioate, boranophosphate, boranophosphorothioate, methyl boranophosphate, methyl boranophosphorothioate, methyl boranophosphonate, methyl boranophosphonothioate, and derivatives thereof; phosphoramidite; phosphoramidate; N3' → P5' phosphoramidate; phosphordiamidate; phosphorothiodiamidate; sulfamate; dimethylenesulfoxide; sulfonate; triazole; oxalyl; carbamate; methyleneimino (MMI); thioacetamido nucleic acid (TANA); derivatives thereof; and any combinations thereof;

and/or

iii) optionally wherein the sugar modification is selected from the group consisting of 2'-O-modified RNA such as 2'-O-alkyl or 2'-O- (substituted)alkyl e.g. 2'-O-methyl, 2'-O-(2-cyanoethyl), 2'-O-(2-methoxy)ethyl (2'-MOE), 2'-O-(2-thiomethyl)ethyl, 2'-O-butyryl, 2'-O-propargyl, 2'-O-allyl, 2'-O-(3-amino)propyl, 2'-O-(3- (dimethylamino)propyl), 2'-O-(2-amino)ethyl, 2'-O-(2-(dimethylamino)ethyl); 2'-deoxy (DNA); 2'-O- (haloalkoxy)methyl (Arai K. et al. Bioorg. Med. Chem. 2011, 21, 6285) e.g. 2'-O-(2- chloroethoxy)methyl (MCEM), 2'-O-(2, 2-dichloroethoxy)methyl (DCEM); 2'-O- alkoxycarbonyl e.g. 2'- O-[2-(methoxycarbonyl)ethyl] (MOCE), 2'-O-[2-(N-methylcarbamoyl)ethyl] (MCE), 2'-O-[2-(N, Ndimethylcarbamoyl)ethyl] (DCME); 2'-halo e.g. 2'-F, FANA (2'-F arabinosyl nucleic acid); carbasugar and azasugar modifications; 3'-O-alkyl e.g. 3'-O-methyl, 3'-O-butyryl, 3'-O-propargyl; and their derivatives. Other sugar modifications include "bridged" or "bicylic" nucleic acid (BNA), e.g. locked nucleic acid (LNA), xylo-LNA, a-L-LNA, β-D-LNA, cEt (2'-O,4'-C constrained ethyl) LNA, cMOEt (2'-O,4'-C constrained methoxyethyl) LNA, ethylene-bridged nucleic acid (ENA), tricycio DNA; unlocked nucleic acid (UNA); cyclohexenyl nucleic acid (CeNA), altriol nucleic acid (ANA), hexitol nucleic acid (HNA), fluorinated HNA (F-HNA), pyranosyl-RNA (p-RNA), 3'-deoxypyranosyl-DNA (p-DNA); morpholino (as e.g. in PMO, PPMO, PMOPlus, PMO-X); and their derivatives and any combination thereof;
and/or

iv) optionally wherein the other modification is selected from the group consisting of peptide-base nucleic acid (PNA), boron modified PNA, pyrrolidine-based oxy-peptide nucleic acid (POPNA), glycol- or glycerol-based nucleic acid (GNA), threose-based nucleic acid (TNA), acyclic threoninol-based nucleic acid (aTNA), oligonucleotides with integrated bases and backbones, pyrrolidine-amide oligonucleotides (POMs), linker (e.g. propane, hexane or polyethyleneglycole), brancher (e.g. symmetrical or asymmetrical brancher), lipid (e.g. palmitate, stearate), peptide or dye-modified oligonucleotides as well as cholesterol- and GalNAc-modified oligonucleotides; and their derivatives and any combination thereof.

[47] The method of any one of items [1] to [27], wherein said molecule of interest is a modified polypeptide comprising one or more modifications selected from the group consisting of phosphorylation, disulfide bonds, glycosylation, acetylation, amidation, gamma-carboxyglutamic acid hydroxylation, methylation, sulfatation, lipids (particularly selected from myristate, palmitate, farnesyl, geranyl-geranyl, a GPI anchor, N-acyl diglyceride), linkers, dyes, and signal sequences.

[48] The method of item [47] wherein said modification is selected from the group consisting of linkers, lipids, sugars, and dyes.

[49] The method of any one of items [1] to [48], wherein said molecule of interest is selected from the group consisting of a compound for research purposes, a commercial compound, an active pharmaceutical ingredient (API), an adjuvant, and an excipient.

[50] The method of any one of items [1] to [49], wherein said molecule of interest is an active pharmaceutical ingredient (API).

[51] The method of any one of items [1] to [49], wherein said method is a method for preparing an active pharmaceutical ingredient (API) for incorporation into a medicament and/or a method for concentrating an active pharmaceutical ingredient (API) for incorporation into a medicament.

[52] The method of any one of items [1] to [51], wherein said method is a method that does not comprise lyophilization.

[53] The method of any one of items [1] to [52], wherein the ultrafiltration membrane has a MWCO in the range between 1 and 150 kDa, in particular between 1 and 100 kDa, particularly between 1 and 30 kDa, especially between 1 and 10 kDa.

[54] The method of item [53], wherein the ultrafiltration membrane has a MWCO in the range between 1 and 100 kDa.

[55] The method of item [53] or [54], wherein the ultrafiltration membrane has a MWCO in the range between 1 and 30 kDa.

[56] The method of any one of items [53] to [55], wherein the ultrafiltration membrane has a MWCO in the range between 1 and 10 kDa.

[57] The method of any one of items [1] to [56], wherein the ultrafiltration membrane has a MWCO in the range between 1 and 7 kDa, such as between 1 and 5 kDa, particularly between 1 and 3 kDa, such of about 2 kDa.

[58] The method of any one of items [1] to [57], wherein the ratio P/R is at least 1.25, particularly at least 1.5, especially at least 2.0, in particular at least 3.0, or at least 5.0, or at least 10.0, or at least 20.0; wherein

P is of the osmolarity of the solution on the permeate side of the ultrafiltration membrane, and
R is the osmolarity of the solution on the retentate side of the ultrafiltration membrane.

[59] The method of any one of items [1], [5], [6] and [8] to [58], wherein - in said step of contacting the permeate side of the ultrafiltration membrane with a solution comprising osmolytes - the ratio P/R is at least 1.25, particularly at least 1.5, especially at least 2.0, in particular at least 3.0, or at least 5.0, or at least 10.0, or at least 20.0; wherein

P is of the osmolarity of the solution on the permeate side of the ultrafiltration membrane, and
R is the osmolarity of the solution on the retentate side of the ultrafiltration membrane.

[60] The method of any one of items [2] to [5], [7] to [57], and [58], wherein the method comprises providing an osmolarity of the solution on the permeate side such that the ratio P/R is at least 1.25, particularly at least 1.5, especially at least 2.0, in particular at least 3.0, or at least 5.0, or at least 10.0, or at least 20.0; wherein

P is of the osmolarity of the solution on the permeate side of the ultrafiltration membrane, and
R is the osmolarity of the solution on the retentate side of the ultrafiltration membrane.

[61] The method of any one of items [58] to [60], wherein said ratio P/R is at least 1.25.

[62] The method of any one of items [58] to [61], wherein said ratio P/R is at least 2.

[63] The method of any one of items [58] to [62], wherein said ratio P/R is at least 5.

[64] The method of any one of items [58] to [63], wherein said ratio P/R is at least 10.

[65] The method of any one of items [58] to [64], wherein said ratio P/R is at least 20.

[66] The method of any one of items [58] to [65], wherein said ratio P/R is at least 1.25 throughout the whole method.

[67] The method of any one of items [58] to [66], wherein said ratio P/R is at least 1.5, especially at least 2.0, in particular at least 3.0, or at least 5.0, or at least 10.0, or at least 20.0 throughout the whole method.

[68] The method of any one of items [1] to [67], wherein said osmolarity of the solution on the permeate side of the ultrafiltration membrane is higher than that on the retentate side of the ultrafiltration after an equilibrium has been reached with respect to small molecules that have passed through the ultrafiltration membrane.

[69] The method of any one of items [1] to [68], wherein said osmolytes are provided in a starting solution comprising said osmolytes.

[70] The method of item [69], wherein the ratio S1/S2 is at least 1.25, particularly at least 1.5, especially at least 2.0, in particular at least 3.0, or at least 5.0, or at least 10.0, or at least 20.0, or at least 30.0; wherein

S1 is the osmolarity of the starting solution comprising said osmolytes, and
S2 is the osmolarity of the starting solution comprising said molecules of interest which is employed in the method.

[71] The method of any one of items [69] to [70], wherein the ratio S1/S2 is at least 2.

[72] The method of any one of items [69] to [71], wherein the ratio S1/S2 is at least 5.

[73] The method of any one of items [69] to [72], wherein the ratio S1/S2 is at least 10.

[74] The method of any one of items [69] to [73], wherein the ratio S1/S2 is at least 30.

[75] The method of any one of items [1] to [74], wherein the osmolarity on the retentate side is calculated by taking into account all osmolytes comprised in the respective solution, and wherein the osmolarity on the permeate side is calculated by only taking into account said osmolytes having a molecular weight that is higher than the MWCO of the ultrafiltration membrane.

[76] The method of any one of items [1] to [74], wherein each osmolarity is calculated by only taking into account osmotically active molecules (including the molecules of interest) that are comprised in the respective solutions, which have a molecular weight that is higher than the MWCO of the ultrafiltration membrane.

[77] The method of any one of items [1] to [76], wherein said osmolytes having a molecular weight that is higher than the MWCO of the ultrafiltration membrane are provided in a starting solution at a concentration of at least 12.5 mM, in particular at least 25 mM, particularly of at least 40 mM, especially of at least 50 mM.

[78] The method of item [77], wherein said osmolytes having a molecular weight that is higher than the MWCO of the ultrafiltration membrane are provided in a starting solution at a concentration of at least 25 mM.

[79] The method of any one of item [77] and [78], wherein said osmolytes having a molecular weight that is higher than the MWCO of the ultrafiltration membrane are provided in a starting solution at a concentration of at least 40 mM.

[80] The method of any one of item [77] to [79], wherein said osmolytes having a molecular weight that is higher than the MWCO of the ultrafiltration membrane are provided in a starting solution at a concentration of at least 50 mM.

[81] The method of any one of items [68] to [80], wherein said osmolytes having a molecular weight that is higher than the MWCO of the ultrafiltration membrane are provided in a starting solution, the molarity of which is at least 2 times the molarity of the starting solution comprising said molecules of interest.

[82] The method of item [81], wherein said osmolytes having a molecular weight that is higher than the MWCO of the ultrafiltration membrane are provided in a starting solution, the molarity of which is at least 5 times the molarity of the starting solution comprising said molecules of interest.

[83] The method of any one of items [1] to [82], wherein the method comprises one or more preceding further steps for ultrafiltrating said molecules of interest, in particular for concentrating said molecules of interest.

[84] The method of item [83] wherein said one or more further steps do not involve the use of osmolytes having a molecular weight that is higher than the MWCO of the ultrafiltration membrane on the permeate side of the ultrafiltration membrane.

[85] The method of any one of items [1], [5], [6] and [8] to [84], wherein said step of contacting the permeate side of the ultrafiltration membrane with a solution comprising osmolytes is preceded by one or more further steps for ultrafiltrating said molecules of interest, in particular for concentrating said molecules of interest.

[86] The method of item [85], wherein said one or more further steps do not involve the use of osmolytes having a molecular weight that is higher than the MWCO of the ultrafiltration membrane on the permeate side of the ultrafiltration membrane.

[87] The method of any one of items [1], [5], [6] and [8] to [86], wherein said step of contacting the permeate side of the ultrafiltration membrane with a solution comprising osmolytes is repeated at least once.

[88] The method of item [87], wherein each repetition involves using a new starting solution comprising osmolytes having a molecular weight that is higher than the MWCO of the ultrafiltration membrane.

[89] The method of any one of items [2] to [5] and [7] to [84], wherein said provision of osmolytes is preceded by one or more steps for ultrafiltrating said molecules of interest, in particular for concentrating said molecules of interest.

[90] The method of item [89], wherein said one or more steps do not involve the use of osmolytes having a molecular weight that is higher than the MWCO of the ultrafiltration membrane on the permeate side of the ultrafiltration membrane.

[91] The method of any one of items [2] to [5], [7] to [84], and [89] to [90] wherein said provision of osmolytes is repeated at least once.

[92] The method of item [91], wherein each repetition involves using a new starting solution comprising osmolytes having a molecular weight that is higher than the MWCO of the ultrafiltration membrane.

[93] The method of any one of the preceding items, wherein the method does not include steps, particularly does not include ultrafiltration steps, that do not involve providing said osmolytes, which have a molecular weight that is higher than the MWCO of the ultrafiltration membrane, on the permeate side of the ultrafiltration membrane.

[94] The method of any one of the preceding items, wherein the starting solution comprising said molecules of interest which is employed in the method has previously been subjected to a concentration step, which is preferably selected from i) a precipitation step and ii) an ultrafiltration method that does not include using said osmolytes, which have a molecular weight that is higher than the MWCO of the ultrafiltration membrane, on the permeate side of the ultrafiltration membrane.

[95] The method of any one of the preceding items, wherein one or more of any method step(s) are repeated.

[96] The method of any one of the preceding items, wherein the method either comprises circulation or flow-through of the solution comprising said osmolytes on the permeate side of the ultrafiltration membrane, preferably wherein the method comprises circulating the solution comprising said osmolytes on the permeate side of the ultrafiltration membrane.

[97] The method of item [96], wherein said circulating involves use of a pump and optionally also the use of a reservoir that is part of the circulation and that initially includes the starting solution comprising said osmolytes.

[98] The method of any one of the preceding items, wherein the phrase "contacting the permeate side of the ultrafiltration membrane with a solution..." is replaced by the term "combining the permeate with a solution".

[99] The method of any one of the preceding items, wherein the method comprises the use of one or more pumps.

[100] The method of any one of the preceding items, wherein the method comprises a step of rinsing the retentate side of the ultrafiltration membrane to increase the yield of the molecules of interest.

[101] The method of any one of the preceding items, wherein the osmolyte is selected from the group consisting of polyethylene glycol (PEG), polyacrylic acid (PAA) sodium salt, polyvinyl pyrrolidone, carboxymethyl cellulose, polyvinyl alcohol, and mixtures thereof, particularly wherein the osmolyte is PEG.

[102] The method of any one of the preceding items, wherein the osmolyte has an average molecular weight of between 1000 and 10,000,000 g/mol, particularly between 1000 and 2,000,000 g/mol, more particularly of between 2000 and 200,000 g/mol.

[103] The method of any one of the preceding items, wherein the osmolyte has an average molecular weight of between 2,000 and 60,000 g/mol, in particular of between 2,000 and 20,000 g/mol, such as of between 4,000 and 10,000 g/mol.

[104] The method of any one of item [1] to [103], wherein the osmolyte, e.g. said PEG, is present at a concentration of at least 12.5 mM, in particular of at least 20 mM, particularly of at least 25 mM, especially of at least 40 mM, especially of at least 50 mM.

[105] The method of any one of item [1] to [104], wherein the osmolyte has a molecular weight that exceeds the MWCO of the ultrafiltration membrane by a factor of at least 1.2, particularly by a factor of at least 1.25, particularly by a factor of at least 1.3, especially by a factor of at least 1.5, in particular by a factor of at least 2.0, more particularly by a factor of at least 3.0, such as by a factor of at least 4.0.

[106] The method of any one of item [1] to [105], wherein the osmolyte essentially does not pass through the ultrafiltration membrane, particularly wherein less than 10% of the osmolyte, preferably less than 5% of the osmolyte, in particular less than 2% of the osmolyte, especially less than 1% of the osmolyte, more particularly less than 0.1% of the osmolyte, more particularly less than 0.01% of the osmolyte, more particularly less than 0.001% of the osmolyte pass through the ultrafiltration membrane.

[107] The method of any one of item [1] to [106], wherein the molecule of interest essentially does not pass through the ultrafiltration membrane, particularly wherein less than 10% of the molecule of interest, preferably less than 5% of the molecule of interest, in particular less than 2% of the molecule of interest, especially less than 1% of the molecule of interest, more particularly less than 0.1% of the molecule of interest, more particularly less than 0.01% of the molecule of interest, more particularly less than 0.001% of the molecule of interest pass through the ultrafiltration membrane.

[108] The method of any one of the preceding items, wherein said method is

    i) a method for enhancing concentrating said solution comprising said molecules of interest;
    ii) a method for improving the efficiency of the ultrafiltration particularly a method for improving the concentration achieved by ultrafiltration;
    iii) a method for enhancing osmotic flow through the ultrafiltration membrane;
    iv) a method for drawing solvent molecules (particularly selected from water, organic solvents and mixtures thereof) from the solution comprising said molecule of interest;
    v) a method for achieving a higher final concentration of the molecule of interest;
    vi) a method for preparing a compound selected from the group consisting of a compound for research purposes, a commercial compound, an active pharmaceutical ingredient (API), an adjuvant, and an excipient;
    vii) a method for preparing an active pharmaceutical ingredient (API), particularly a method for preparing a medicament;
    viii) an ultrafiltration/diafiltration method;
    ix) a method that allows concentrating said solution comprising the molecule of interest to a concentration of at least 150 mg/ml; particularly of at least 200 mg/ml; particularly of at least 250 mg/ml; particularly of at least 300 mg/ml; particularly of at least 350 mg/ml; especially of at least 400 mg/ml;
    x) a method for avoiding lyophilization;
    xi) a method for improving the efficiency of subsequent lyophilization;
    and/or
    xii) a method for enhancing flux through the ultrafiltration membrane.

[109] The method of item [108], wherein said method is a method for enhancing concentrating said solution comprising said molecules of interest.

[110] The method of any one of items [108] to [109], wherein said method is a method for improving the efficiency of the ultrafiltration.

[111] The method of any one of items [108] to [110], wherein said method is a method for enhancing osmotic flow through the ultrafiltration membrane.

[112] The method of any one of items [108] to [111], wherein said method is a method for drawing solvent molecules (particularly selected from water, organic solvents and mixtures thereof) from the solution comprising said molecule of interest.

[113] The method of any one of items [108] to [112], wherein said method is a method for achieving a higher final concentration of the molecule of interest.

[114] The method of any one of items [108] to [113], wherein said method is a method for preparing a compound selected from the group consisting of a compound for research purposes, a commercial compound, an active pharmaceutical ingredient (API), an adjuvant, and an excipient, and particularly for preparing an active pharmaceutical ingredient (API).

[115] The method of any one of items [108] to [114], wherein said method is a method for preparing a medicament.

[116] The method of any one of items [108] to [115], wherein said method is a tangential flow filtration method, particularly wherein said method is an ultrafiltration/diafiltration method.

[117] The method of any one of items [108] to [116], wherein said method is a method that allows concentrating said solution comprising the molecule of interest to a concentration of at least 150 mg/ml; particularly of at least 200 mg/ml; particularly of at least 250 mg/ml; particularly of at least 300 mg/ml; particularly of at least 350 mg/ml; especially of at least 400 mg/ml.

[118] The method of any one of items [108] to [117], wherein said method is a method for avoiding lyophilization or a method for improving the efficiency of subsequent lyophilization.

[119] The method of any one of items [108] to [118], wherein said method is a method for enhancing flux through the ultrafiltration membrane.

[120] Use of osmolytes in an method for ultrafiltrating a solution comprising molecules of interest, the method comprising a step of contacting the permeate side of the ultrafiltration membrane with a solution comprising said osmolytes, wherein said osmolytes have a molecular weight that is higher than the MWCO of the ultrafiltration membrane, and wherein the solution on the permeate side of the ultrafiltration membrane has a higher osmolarity than the solution on the retentate side of the ultrafiltration membrane;
particularly wherein said use is further defined in accordance with any of items [1] to [119].

[121] The use of item [120], wherein said method for ultrafiltrating is further characterized as defined in any one of items [1], [5], [6], [8] to [88], and [93] to [119].

[122] Use of osmolytes for improving a method for ultrafiltrating a solution comprising molecules of interest, wherein said osmolytes have a molecular weight that is higher than the MWCO of the ultrafiltration membrane, and wherein said osmolytes are provided on the permeate side of the ultrafiltration membrane in order to improve ultrafiltration;
particularly wherein said use is further defined in accordance with any of items [1] to [119].

[123] The use of item [122], wherein said method for ultrafiltrating is further characterized as defined in any one of items [2] to [5], [7] to [84], [89] to [119].

[124] The use of any one of items [120] to [123], wherein said method for ultrafiltrating is a method for concentrating a solution comprising molecules of interest via ultrafiltration.

[125] The use of any one of items [120] to [124], wherein said use is for

    i) enhancing concentrating said solution comprising molecules of interest;
    ii) improving the efficiency of the ultrafiltration;
    iii) enhancing osmotic flow through the ultrafiltration membrane;
    iv) drawing solvent molecules (particularly selected from water, organic solvents and mixtures thereof) from the solution comprising said molecule of interest;
    v) achieving a higher final concentration of the molecule of interest;
    vi) for preparing a compound selected from the group consisting of a compound for research purposes, a commercial compound, an active pharmaceutical ingredient (API), an adjuvant, and an excipient;
    vii) for preparing an active pharmaceutical ingredient (API), particularly for preparing a medicament;
    viii) ultrafiltration/diafiltration of the molecule of interest;
    ix) concentrating said solution comprising the molecule of interest to a concentration of at least 150 mg/ml; particularly of at least 200 mg/ml; particularly of at least 250 mg/ml; particularly of at least 300 mg/ml; particularly of at least 350 mg/ml; especially of at least 400 mg/ml;
    x) avoiding lyophilization;
    xi) improving the efficiency of subsequent lyophilization;
    and/or
    xii) enhancing flux through the ultrafiltration membrane.

[126] The use of item [125], wherein said use is for enhancing concentrating said solution comprising said molecules of interest.

[127] The use of any one of items [125] to [126], wherein said use is for improving the efficiency of the ultrafiltration.

[128] The method of any one of items [125] to [126], wherein said use is for enhancing osmotic flow through the ultrafiltration membrane.

[129] The use of any one of items [125] to [126], wherein said use is for drawing solvent molecules (particularly selected from water, organic solvents and mixtures thereof) from the solution comprising said molecule of interest.

[130] The use of any one of items [125] to [126], wherein said use is for achieving a higher final concentration of the molecule of interest.

[131] The use of any one of items [125] to [126], wherein said use is for preparing a compound selected from the group consisting of a compound for research purposes, a commercial compound, an active pharmaceutical ingredient (API), an adjuvant, and an excipient.

[132] The use of any one of items [125] to [126], wherein said use is for preparing an active pharmaceutical ingredient, particularly for preparing a medicament.

[133] The use of any one of items [125] to [126], wherein said use is in an ultrafiltration/diafiltration method.

[134] The use of any one of items [125] to [126], wherein said use allows concentrating said solution comprising the molecule of interest to a concentration of at least 150 mg/ml; particularly of at least 200 mg/ml; particularly of at least 250 mg/ml; particularly of at least 300 mg/ml; particularly of at least 350 mg/ml; especially of at least 400 mg/ml.

[135] The use of any one of items [125] to [126], wherein said use is for avoiding lyophilization or for improving the efficiency of subsequent lyophilization.

[136] The use of any one of items [125] to [126], wherein said use is for enhancing flux through the ultrafiltration membrane.

[137] An ultrafiltration device for filtrating, particularly for concentrating, a solution comprising molecules of interest, characterized in that the device comprises

i) means for contacting the permeate side of the ultrafiltration membrane with a solution comprising osmolytes having a molecular weight that is higher than the MWCO of the ultrafiltration membrane, to increase flux through the ultrafiltration membrane;
and/or

ii) means for combining the ultrafiltration permeate with a solution comprising osmotically active molecules having a molecular weight that is higher than the MWCO of the ultrafiltration membrane, and (optionally) means for circulating the resulting solution on the permeate side of the ultrafiltration membrane.

[138] The ultrafiltration device of item [137], wherein (all of) said means are characterized in that they do not return the permeate to the retentate side of the ultrafiltration membrane.

[139] The ultrafiltration device of item [137] or [138], wherein said means for circulation comprise a pump and a reservoir for the starting solution comprising said osmolytes.

[140] The ultrafiltration device of any one of items [137] to [139], wherein the device is configured to operate i) in flow-through mode, wherein the retentate is not recirculated on the retentate side of the membrane, or ii) in recirculation mode, wherein the retentate is recirculated on the retentate side of the membrane, and preferably to operate as in item ii).

[141] The ultrafiltration device of any one of items [137] to [140], wherein the device is configured to operate i) in flow-through mode, wherein the permeate is not recirculated on the permeate side of the membrane, or ii) in recirculation mode, wherein the permeate is recirculated on the permeate side of the membrane, and preferably to operate as in item ii).

[142] The ultrafiltration device of any one of items [137] to [140], wherein the device comprises said starting solution comprising said osmolytes, particularly wherein said device and/or said starting solution is further defined as in any of the above items.

DESCRIPTION OF THE FIGURES

Figure 1: Schematic Drawing of an exemplary experimental setup according to the present invention, according to which the osmolyte solution is recycled. Only the main circulation elements are shown.

Figure 2: Schematic Drawing of a more specific exemplary experimental setup, according to which the osmolyte solution is recycled.

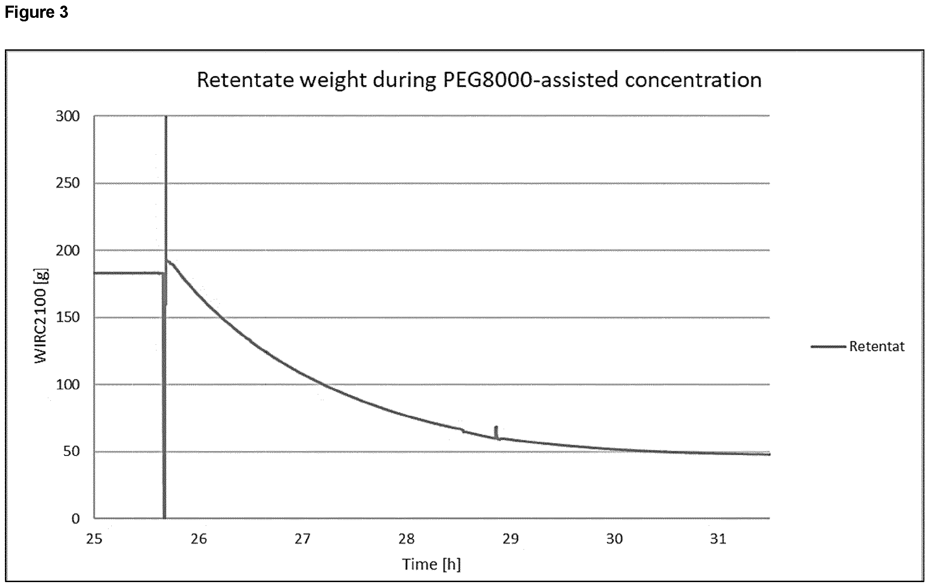

Figure 3: Exemplary recording of the retentate weight (WIRC2100[g]) during PEG8000-assisted concentration. The weight of the retentate (solution) has been monitored over time during concentration according to a method of the invention.

Figure 4: Schematic drawing of a more specific exemplary experimental setup utilizing a flow-through (instead of recycling, see Figures 1 and 2) concept for using the osmolyte

DETAILED DESCRIPTION

In a first aspect, the present invention relates to a method for ultrafiltrating a solution comprising molecules of interest, the method comprising a step of contacting the permeate side of the ultrafiltration membrane with a solution comprising osmolytes having a molecular weight that is higher than the MWCO of the ultrafiltration membrane, wherein the solution on the permeate side of the ultrafiltration membrane has a higher osmolarity than the solution on the retentate side of the ultrafiltration membrane.
In a second aspect, the present invention relates to a method for ultrafiltrating a solution comprising molecules of interest, characterized in that it comprises providing a higher osmolarity on the permeate side of the ultrafiltration membrane than on the retentate side of the ultrafiltration membrane by providing osmolytes, which have a molecular weight that is higher than the MWCO of the ultrafiltration membrane, on the permeate side of the ultrafiltration membrane.
In a third aspect, the present invention relates to a method for ultrafiltrating a solution comprising molecules of interest, characterized in that it comprises providing osmolytes, which have a molecular weight that is higher than the MWCO of the ultrafiltration membrane, on the permeate side of the ultrafiltration membrane, in order to improve ultrafiltration.
In a fourth aspect, the present invention relates to a method for ultrafiltrating a solution comprising molecules of interest, characterized in that it comprises generating an osmotic flow through the ultrafiltration membrane, wherein said osmotic flow is generated by osmolytes, which have a molecular weight that is higher than the MWCO of the ultrafiltration membrane, provided on the permeate side of the ultrafiltration membrane.
Generally herein, in preferred embodiments, the method of any one of the preceding aspects is a method for concentrating a solution comprising molecules of interest via ultrafiltration.
Consequently, in a further aspect (fifth aspect), the present invention relates to a method for preparing a concentrated solution of molecules of interest from a starting solution of said molecules of interest, wherein the method employs ultrafiltration, wherein the method comprises a step of contacting the permeate side of the ultrafiltration membrane with a solution comprising osmolytes having a molecular weight that is higher than the MWCO of the ultrafiltration membrane, wherein the solution on the permeate side of the ultrafiltration membrane has a higher osmolarity than the solution on the retentate side of the ultrafiltration membrane.
Similarly, in a further aspect (sixth aspect), the present invention relates to a method for preparing a concentrated solution of molecules of interest from a starting solution of said molecules of interest, wherein the method employs ultrafiltration and wherein the method is characterized in that concentrating comprises providing osmolytes on the permeate side of the ultrafiltration membrane which osmolytes have a molecular weight that is higher than the MWCO of the ultrafiltration membrane.
As to the methods of the present invention, "methods for ultrafiltrating" are generally well known to the skilled reader as also indicated in the introduction herein and correspond to the understanding in the art. In the present invention, the term is used interchangeably with "ultrafiltration methods". Ultrafiltration methods of the present invention include methods for direct filtration and tangential filtration, the latter being preferred in context with the invention. Ultrafiltration methods of the present invention include methods of ultrafiltration/diafiltration, a variant of ultrafiltration also designated herein as "UF/DF", without limitation.

Generally, there are two main modes for carrying out ultrafiltration, namely direct filtration (also called "dead-end filtration") and tangential flow filtration ("TFF"), the latter of which is often also referred to as cross flow filtration ("CFF"). In view of that, also for the purposes of the present description, the terms TFF and CFF may be used interchangeably herein.

In any case, in the present invention, tangential flow filtration (TFF) is the preferred type of ultrafiltration.

A core piece of any ultrafiltration device is the ultrafiltration membrane, which is not particularly limited herein and may significantly vary in form, shape and type. In any case, the "ultrafiltration membrane" herein is characterized as a semipermeable membrane comprising two sides, namely the "retentate side" and the "permeate side". As used herein the "retentate side of the ultrafiltration membrane" is the side, on which molecules of interest are retained.

As used herein "flux through the ultrafiltration membrane" refers to the passing through of small molecules through the ultrafiltration membrane, the sizes of which are smaller than the size of the pores of the membrane. The term includes the flux of water molecules and low molecular weight compounds, non-limiting examples of which are salt molecules and organic solvent molecules. In the methods of the invention, said flux may be promoted by the pressure generally employed in ultrafiltration and additionally by the use of the osmolytes.

In any case, in certain embodiments herein, the ultrafiltration methods of the invention are carried out without applying an additional transmembrane pressure (TMP).

Ultrafiltration may also involve the use of pressure to generate and maintain flux through the ultrafiltration membrane. Consequently, in alternative general embodiments herein, the ultrafiltration methods of the invention are carried out with applying an additional transmembrane pressure (TMP). In particular embodiments, said TMP may be applied throughout the method, whereas in other embodiments, said TMP is applied only during part of the methods.

Moreover, when using tangential flow filtration (TFF) in line with preferred embodiments of the invention, there is an advantage of having a flow direction of the retentate along the membrane, which e.g. allows enhanced efficiency, much less clogging of the membrane, etc. as the skilled reader will readily appreciate.

In any case, all such ways of carrying out ultrafiltration are generally well known to the skilled person as also described elsewhere herein.

Generally, as used herein the term "osmolarity" is used as it is understood in the art and may interchangeably be used with "osmotic concentration". It is usually used herein with reference to a given solution, such as the solution that is in contact with the ultrafiltration membrane on the retentate side or the solution that is in contact with the ultrafiltration on the permeate side. Consequently, if a "solution on the permeate side of the ultrafiltration membrane has a higher osmolarity than the solution on the retentate side of the ultrafiltration membrane" (and the like) this is believed to trigger an osmotic flow through the ultrafiltration membrane, which contributes to the above flux.

Generally, the skilled reader will readily be in a position to determine whether or not the solution on the permeate side of the ultrafiltration membrane has a higher osmolarity than the solution on the retentate side of the ultrafiltration membrane.

Osmolarity is easily calculated by determining the number of solute particles per 1 L of solvent. For example, for a solution of 1 mol/l NaCl, the osmolarity is 2 osmol/l due to the dissolution of NaCl into $Na^+$ cations and $Cl^-$ anions.

As opposed to osmolarity, osmolality is calculated by determining the number of solute particles per 1 kg of solvent, and hence is independent from the respective temperature. In general alternative embodiments herein, the term "osmolarity" is replaced by "osmolality".

Both osmolarity and osmolality are standard measures of concentration of particles dissolved in a solution, which are only based on the number of solute particles, not on the nature, size and shape of the respective molecules. In principle the aqueous solution of higher osmolarity / osmolality triggers an osmotic flow through the ultrafiltration membrane from the side of lower osmolarity until an equilibrium is reached. Because of the (selective) permeability of an ultrafiltration membrane, only molecules of smaller size (e.g. ions or other smaller molecules) can permeate and influence osmolarity.

The total osmolality of aqueous solutions may be determined by comparative measurements, e.g. of the freezing points of pure water in relation to the aqueous solutions of interest. Whereas water has a freezing point of 0°C, a solution with saline concentration of 1 Osmol/kg has a freezing point of -1.858°C (a phenomenon also called freezing point depression). In one embodiment the osmolality (or osmolarity) is determined as described in US Pharmacopeia 36 <785>. In another embodiment the osmolality (or osmolarity) is determined as described in European Pharmacopeia 7.3, section 2.2.35.

In any case, a solution on the permeate side of the ultrafiltration membrane does not need to have a higher osmolarity than the solution on the retentate side of the ultrafiltration membrane throughout the whole methods of the invention. Rather, in certain preferred embodiments of the methods and uses of the invention, a "solution on the permeate side of the ultrafiltration membrane has a higher osmolarity than the solution on the retentate side of the ultrafiltration membrane" e.g. at least at a time where there would no longer be a net flow of small molecules through the ultrafiltration membrane in the absence of said osmolytes. In any case, and depending on the particular starting solutions employed, there may initially be a higher osmolarity on the retentate side of the membrane.

Moreover, in context with the present invention the term "osmolyte", which may interchangeably be used herein with the phrase "osmotically active molecule", generally refers to molecules that contribute to the osmolarity of a given solution. Most of the times herein (and even though also the "molecules of interest" are strictly speaking osmolytes as well) the term "osmolytes" refers to osmolytes on the permeate side of the ultrafiltration membrane, as will also become readily apparent from the respective contexts herein.

In any case, as the skilled reader will readily understand, osmolytes having a molecular weight that is higher than the MWCO ("molecular weight cut-off") of the ultrafiltration membrane are osmotically active in that they are suitable to trigger an osmotic flow towards the side of the membrane, on which said molecules are present.

Without intending to be bound by theory, said osmotic flow is considered to be the higher the higher the concentration of said osmolytes is and the lower the concentration of other osmotically active molecules is on the opposite side (retentate side) of the ultrafiltration membrane. Consequently, and particularly at a time where small molecules no longer pass through the ultrafiltration membrane, said osmotic flow is considered to be the higher the greater the difference in the concentration of the molecules of interest and said osmolytes is.

Furthermore, "improving ... ultrafiltration", an "improvement of ultrafiltration" and suchlike terms are used interchangeably herein and may refer to various kinds of improvement, such as an enhanced flux through the ultrafiltration membrane, an enhanced level of concentrating the molecules of interest, and/or an enhanced separation of undesired molecules (which are able to pass through the membrane) from the solution comprising the molecules of interest.

With respect to the methods of the present invention, it will further be readily understood by the person skilled in the art reading the present claims and description that osmotic phenomena as the ones relied upon in the present invention require the presence of multiple molecules such as multiple "osmolytes" or "osmotically active molecules", respectively - and that solutions of molecules of interest generally comprise a multitude of molecules of interest. Accordingly, whenever reference is made to the singular form of such molecules, said singular form likewise includes the plural forms without explicit indication.

As used herein, the phrase according to which the osmolyte(s) "have a molecular weight that is higher than the MWCO of the ultrafiltration membrane" and suchlike terms are readily understood by the skilled reader, wherein the "MWCO" is an abbreviation for the "molecular weight cut-off" value of the ultrafiltration membrane.

Preferably, both said molecular weight and the MWCO are given in kDa, such that they are easily comparable.

As will be readily appreciated by the skilled artisan, a MWCO preferably designates the lowest molecular weight of molecules, which are retained to at least 90%.

According to preferred embodiments herein, the osmolyte has a molecular weight that exceeds the MWCO of the ultrafiltration membrane by a factor of at least 1.2, particularly by a factor of at least 1.25, particularly by a factor of at least 1.3, especially by a factor of at least 1.5, in particular by a factor of at least 2.0, more particularly by a factor of at least 3.0, such as by a factor of at least 4.0.

In certain general alternative embodiments herein, the osmolyte(s) have an "average molecular weight" that is higher than the MWCO of the ultrafiltration membrane, wherein further particular embodiments thereof correspond to those depicted herein for the "molecular weight"

According to corresponding preferred embodiments herein, the osmolyte has an average molecular weight that exceeds the MWCO of the ultrafiltration membrane by a factor of at least 1.3, particularly by a factor of at least 1.35, particularly by a factor of at least 1.4, especially by a factor of at least 1.5, in particular by a factor of at least 2.0, more particularly by a factor of at least 3.0, such as by a factor of at least 4.0.

Moreover, in a further set of particular embodiments, particularly where retaining the molecules of interest and osmolytes on the respective desired sides of the ultrafiltration membrane is of particular interest herein, it is preferable to use a MWCO that is significantly below the size of the respective molecules, such as 5/8, 1/2, 1/4, 1/5, 1/6, 1/7 or even 1/10 of the size of the respective molecules.

In particular, in certain embodiments, the MWCO is selected to correspond to 5/8 of the value of the molecular weight of the osmolytes. Preferably, the MWCO is selected to correspond to 1/2 of the value of the molecular weight of the osmolytes. In further non-limiting embodiments, the MWCO is selected to correspond to 1/4 of the value of the molecular weight of the osmolytes. In further non-limiting embodiments, the MWCO is selected to correspond to 1/5 of the value of the molecular weight of the osmolytes. In further non-limiting embodiments, the MWCO is selected to correspond to 1/6 of the value of the molecular weight of the osmolytes, and so on.

Likewise, preferably, the MWCO is selected to correspond to 1/2 of the value of the molecular weight of the molecules of interest. In further non-limiting embodiments, the MWCO is selected to correspond to 1/4 of the value of the molecular weight of the molecules of interest. In further non-limiting embodiments, the MWCO is selected to correspond to 1/5 of the value of the molecular weight of the molecules of interest. In further non-limiting embodiments, the MWCO is selected to correspond to 1/6 of the value of the molecular weight of the molecules of interest, and so on.

In context with the present invention, ultrafiltration methods may generally be employed for concentrating respective solutions comprising molecules of interest, whereas a "method for concentrating a solution comprising molecules of interest via ultrafiltration" and respective phrases indicates that the initial concentration of said molecules of interest is increased by said methods.

In certain embodiments, the methods of the invention allow concentrating said molecules of interest to a concentration of at least 100 mg/ml. Preferably, the methods allow concentrating said molecules of interest to a concentration of at least 150 mg/ml. In further preferred embodiments, the methods allow concentrating said molecules of interest to a concentration of at least 200 mg/ml. In particular embodiments, the methods allow concentrating said molecules of interest to a concentration of at least 250 mg/ml, such as to a concentration of at least 300 mg/ml. In particular embodiments, the methods allow concentrating said molecules of interest to a concentration of at least 350 mg/ml, such as to a concentration of at least 400 mg/ml.

In further particular embodiments - particularly in the context of polypeptides as the molecules of interest, the methods allow concentrating said molecules of interest to a concentration of at least 500 mg/ml, preferably of at least 750 mg/ml, such as of up to 1000 mg/ml.

As the person skilled in the art will readily appreciate, absolute values to be achieved depend on the particular molecule of interest - with higher absolute values being generally more easily achieved in the context of polypeptides as compared to polynucleotides.

In preferred embodiments herein, however, a method of the invention is characterized in that the method allows concentrating given molecules of interest to a concentration that is significantly higher than the concentration achievable with the same type of ultrafiltration (preferably TFF or CFF, respectively) not using said osmolytes - whereas the respective difference may be referred to as an "improvement of the concentration" - and wherein said improvement may be indicated by a certain percentage value. In particular embodiments, a method of the inventions achieves an improvement of the concentration of at least 10% (in other words: the concentration achieved by using said osmolytes is at least 10% higher than it would be without using said osmolytes). In further preferred embodiments, said improvement is at least 15%, preferably at least 20%, preferably at least 25%, such as at least 30% or even at least 50%. In other words, respective embodiments may be described herein as "improving ultrafiltration by at least 10%, 15%, ..." and suchlike.

With respect to the term "starting solution" as used herein either in context with the solution comprising the molecules of interest or in context with the solution comprising the osmolytes, the skilled reader will appreciate that same refers to the respective solutions that are initially applied in the respective methods. Here, the skilled person will further appreciate that the present methods may or may not include further (ultrafiltration or other) steps depending on the respective embodiments. Accordingly, a "starting solution" refers to the respective initial solutions used in the respective embodiments - irrespective from any prior processing e.g. of the solution comprising the molecules of interest. Furthermore, it will be appreciated that methods for ultrafiltration are continuous processes due to the flux through the ultrafiltration membrane. Hence, the composition of the respective starting solutions usually does not remain constant due to the process of ultrafiltration. In case of a starting solution comprising said molecules of interest, the contents may e.g. change in dependence on (further) solution(s) introduced on the retentate side of the membrane and/or by circulation of the solution on said side.

Likewise, in case of a starting solution comprising said osmolytes, the contents are expected to change due to the combining of said solution with the emerging eluate and/or by circulation of the solution on the permeate side of the membrane. With respect to the latter solution, however, the composition may remain almost constant by employing a respective high volume of said solution such that dilution is negligible for the purposes of the method.

As also depicted elsewhere herein, in alternative embodiments herein, the solution on the permeate side of the membrane is not circulated. Instead, the solution on the permeate side of the membrane is contacted with the ultrafiltration membrane in flow-through mode. As the skilled reader will appreciate, the latter variant allows continuously contacting the membrane with the same starting solution comprising said osmolytes throughout the method.

Generally, both the nature of said molecules of interest in context with the invention and their sizes are not particularly limited, as will be readily appreciated by the skilled reader, who is aware of the types of molecules that may generally be subjected to ultrafiltration and of respective purposes for doing so. That is, generally herein, the molecules of interest preferably have a molecular weight of between 0.5 kDa and 1000 kDa - and may even have a molecular weight of up to 1500 kDa or even 1800 kDa (such as in case of mRNA having a length of about 6000 nucleotides).

Generally herein, the term "molecules of interest" and suchlike terms are understood as and may be interchangeably used with the term "compounds of interest", such that further embodiments are envisaged herein, in which the term "molecules" is replaced by the term "compounds"

In particularly preferred embodiments, said molecules of interest herein have a molecular weight of from 2 kDa to 200 kDa, such as from 10 kDa to 150 kDa.

Generally herein, the molecules of interest may or may not be modified. Accordingly, in one set of general embodiments, the molecules of interest are not modified.

In a further set of general embodiments, the molecules of interest are modified.

Here, potential modifications for the respective molecules of interest are readily apparent to the person skilled in the art and are not particularly limited.

According to preferred non-limiting embodiments of the methods of the invention said molecules of interest are oligonucleotides or polypeptides. As pointed above, any of those may be modified.

Generally, in the present invention, any modified molecules may be biologically (such as in vivo in cells), chemically, enzymatically or chemo-enzymatically modified.

In preferred embodiments according to the invention, modified molecules herein are chemically modified.

Consequently, in a first set of particularly preferred embodiments, the molecules of interest in context with the invention are oligonucleotides. As depicted above, generally, said oligonucleotides may or may not be modified. Thus, further embodiments to the particular embodiments for oligonucleotides described below are envisaged herein, in which respective oligonucleotides are modified. Preferred ways of modifications are described elsewhere herein.

Oligonucleotides are well known to the person skilled in the art as DNA oligomers or RNA oligomers, which have a wide range of technical applications in many fields, and which may be biologically (such as in vivo in cells), chemically, enzymatically or chemo-enzymatically synthesized and/or modified as desired. In particular, oligonucleotides used in context of the invention may be suitable for medical applications. In general, oligonucleotides herein may be relatively short.

In any case, in certain general embodiments, the molecule of interest is ssDNA (single stranded DNA). In other embodiments, the molecule of interest is dsDNA (double stranded DNA). Likewise, in certain general embodiments, the molecule of interest is ssRNA (single stranded RNA). In other embodiments, the molecule of interest is dsRNA (double stranded RNA). In even further general embodiments, the molecule of interest is a heteroduplex of ssRNA and ssDNA.

Besides, in further embodiments, the molecule of interest is a ribozyme, which are well known as "RNA enzymes" or "RNAzymes", i.e. RNA molecules that catalyze specific biochemical reactions.

Besides, in further embodiments, the molecules of interest are "DNAzymes".

In particular embodiments, the molecule of interest is siRNA (small interfering RNA), which is also known as "short interfering RNA" or "silencing RNA", which typically is double stranded RNA and which can typically be used to silence expression of a given target gene via RNA interference. siRNA may or may not be modified and may or may not contain non RNA elements such as DNA, LNA or GNA. In non-limiting exemplary embodiments, an siRNA is a double stranded RNA with a length of 20-24 base pairs. Generally herein, particularly also in the case of siRNA, a given siRNA may either be subjected to the methods of the invention as a whole - or the individual single strands thereof may be separately subjected to the methods of the invention.

In other embodiments, the molecule of interest is DsiRNA ("dicer-substrate short interfering RNAs"), which are well known to the skilled person as Dicer substrates which are cleaved / processed to result in siRNAs.

In other embodiments, the molecule of interest is shRNA ("short hairpin RNA"), which is also known as "small hairpin RNA", and which is typically known as an artificial RNA molecule comprising a hairpin turn which can be used to silence expression of a target gene via RNA interference.

In even further embodiments, the molecule of interest is miRNA (micro RNA), which is known as small single stranded non coding RNA that may e.g. also work in RNA silencing or in post-transcriptional regulation of gene expression.

In addition, according to other embodiments, the molecule of interest is anti-miRNA, which may be used to counteract and/or neutralize miRNA function.

In even further embodiments, the molecule of interest is mRNA (messenger RNA), which is known as small single stranded coding RNA that has recently e.g. gained particular relevance in the connection with mRNA vaccines.

In even further embodiments, the molecule of interest is rRNA (ribosomal RNA).

In even further embodiments, the molecule of interest is tRNA (transfer RNA).

In even further embodiments, the molecule of interest is sgRNA (single guide RNA).

In certain embodiments, the molecule of interest is a plasmid.

Besides, in other preferred embodiments, the molecule of interest is an antisense oligonucleotide. Antisense oligonucleotides are well known e.g. as single strands of DNA or RNA that are complementary to a chosen sequence and that can e.g. be used to target specific complementary sequences.

In line with general preferred embodiments, the antisense oligonucleotide may be modified. In particular embodiments, the antisense oligonucleotide is modified with or as 2'OMe, 2'MOE, LNA, constrained ethyl nucleic acids (cEt), thioates or as a so-called Gapmer (i.e. a mixed form of DNA and the modifications).

In some non-limiting embodiments, said oligonucleotides have a length of from 10 to 100 nucleotides, particularly of from 15 to 50 nucleotides, especially of from 20 to 30 nucleotides.

In particular embodiments herein, the molecules of interest are oligonucleotides comprising of from 10 to 300 nucleotides, particularly of from 14 to 130 nucleotides, especially of from 16 to 110 nucleotides.

Accordingly, in certain embodiments herein, the molecules of interest are oligonucleotides having a molecular weight of from 3 to 90 kDa, particularly of from 4 to 39 kDa, especially of from 5 to 33 kDa.

In further embodiments herein, the molecules of interest are oligonucleotides comprising of from 10 to 6000 nucleotides (e.g. in case of mRNA as the molecule of interest).

Particular examples of embodiments herein of (therapeutic) oligonucleotides that may advantageously be employed in the methods of the present invention include, but are not limited to Fomivirsen (Vitravene), Pegaptanib sodium (Macugen), Mipomersen (Kynamro), Defibrotide (Defitelio), Eteplirsen (Exondys 51), Nusinersen (Spinraza), Hepatitis B surface antigen (Heplisav-B), Patisiran (Onpattro), Inotersen (Tegsedi), volanesorsen (Waylivra), Givosiran (Givlaari) and Golodirsen (Vyondys53). In the above list, the names in brackets correspond to the therapeutic names, whereas the INN names are given in front of said molecules.

Computational methods for calculating the molecular weight of a given oligonucleotide having a given sequence are readily available (e.g. online) to the person skilled in the art.

In embodiments of the present invention, generally, the oligonucleotides may also be a mixture of oligonucleotides. In one alternative, said oligonucleotides are a mixture of any of the individual types of oligonucleotides defined herein. Hence, in an alternative embodiments, the oligonucleotide is a double stranded oligonucleotide, which is a mixture of two particular oligonucleotides, alternatively of more than two different oligonucleotides. In further non-limiting alternative embodiments, the oligonucleotides may also be a mixture of oligonucleotides of the same type of oligonucleotide defined herein, such as a mixture of various RNAs or suchlike.

In particular embodiments herein, said molecule of interest is an oligonucleotide comprising one or more modifications selected from the group consisting of methylation, carboxymethylation, acetylation, methoxyaminomethylation, methoxycarbonylation, thiolation, adenylation, polyadenylation, base or backbone modifications, respectively (such as that the oligonucleotide is selected from GNA, TNA, FNA), sugar modifications (such as by GalNac), linkers, lipids, peptides, and dyes.

Consequently, the modified oligonucleotides herein may also contain elements that are different from nucleotides, such as "artificial bases" attached to a different chemical backbone than in regular nucleotides. Accordingly, in particular embodiments, the (modified) oligonucleotides in context with the invention are selected from the group consisting of GNA, TNA, and FNA.

In particularly preferred embodiments, said one or more modifications are independently selected from base modifications, backbone modifications, sugar modifications, and other modifications.

In the present invention base modifications are preferably selected from the group consisting of hypoxanthine, inosine, 8-oxo-adenine, 7-substituted derivatives thereof, dihydrouracil, pseudouracil, 2-thiouracil, 4-thiouracil, 5-aminouracil, 5-(C1-C6)-alkyluracil, 5-methyluracil, 5-(C2-C6)-alkenyluracil, 5-(C2-C6)-alkynyluracil, 5-(hydroxymethyl)uracil, 5-chlorouracil, 5-fluorouracil, 5-bromouracil, 5-hydroxycytosine, 5-(C1-C6)-alkylcytosine, 5-methylcytosine, 5-(C2-C6)-alkenylcytosine, 5-(C2-C6)-alkynylcytosine, 5-chlorocytosine, 5-fluorocytosine, 5-bromocytosine, N2-dimethylguanine, 7-deazaguanine, 8-azaguanine, 7-deaza-7-substituted guanine, 7-deaza-7-(C2-C6)alkynylguanine, 7-deaza-8-substituted guanine, 8-hydroxyguanine, 6-thioguanine, 8-oxoguanine, 2-aminopurine, 2-amino-6-chloropurine, 2,4-diaminopurine, 2,6-diaminopurine, 8-azapurine, substituted 7-deazapurine, 7-deaza-7-substituted purine, 7-deaza-8-substituted purine, hydrogen (abasic residue), and any combination thereof.

In the present invention backbone modifications are preferably selected from the group consisting of modified versions of the phosphodiester present in DNA or RNA, preferably selected from phosphorothioate (PS), phosphorodithioate (PS2), phosphonoacetate (PACE), phosphonoacetamide (PACA), thiophosphonoacetate, thiophosphonoacetamide, phosphorothioate prodrug, H-phosphonate, methyl phosphonate, methyl phosphonothioate, methyl phosphate, methyl phosphorothioate, ethyl phosphate, ethyl phosphorothioate, boranophosphate, boranophosphorothioate, methyl boranophosphate, methyl boranophosphorothioate, methyl boranophosphonate, methyl boranophosphonothioate, and derivatives thereof; phosphoramidite; phosphoramidate; N3' → P5' phosphoramidate; phosphordiamidate; phosphorothiodiamidate; sulfamate; dimethylenesulfoxide; sulfonate; triazole; oxalyl; carbamate; methyleneimino (MMI); thioacetamido nucleic acid (TANA); derivatives thereof; and any combinations thereof.

In the present invention sugar modifications are preferably selected from the group consisting of a modified version of the ribosyl moiety, such as 2'-O-modified RNA such as 2'-O-alkyl or 2'-O- (substituted)alkyl e.g. 2'-O-methyl, 2'-O-(2-cyanoethyl), 2'-O-(2-methoxy)ethyl (2'-MOE), 2'-O-(2- thiomethyl)ethyl, 2'-O-butyryl, 2'-O-propargyl, 2'-O-allyl, 2'-O-(3-amino)propyl, 2'-O-(3- (dimethylamino)propyl), 2'-O-(2-amino)ethyl, 2'-O-(2-(dimethylamino)ethyl); 2'-deoxy (DNA); 2'-O-(haloalkoxy)methyl (Arai K. et al. Bioorg. Med. Chem. 2011, 21, 6285) e.g. 2'-O-(2-chloroethoxy)methyl (MCEM), 2'-O-(2, 2-dichloroethoxy)methyl (DCEM); 2'-O-alkoxycarbonyl e.g. 2'- O-[2-(methoxycarbonyl)ethyl] (MOCE), 2'-O-[2-(N-methylcarbamoyl)ethyl] (MCE), 2'-O-[2-(N, Ndimethylcarbamoyl)ethyl] (DCME); 2'-halo e.g. 2'-F, FANA (2'-F arabinosyl nucleic acid); carbasugar and azasugar modifications; 3'-O-alkyl e.g. 3'-O-methyl, 3'-O-butyryl, 3'-O-propargyl; and their derivatives. Other sugar modifications include "bridged" or "bicylic" nucleic acid (BNA), e.g. locked nucleic acid (LNA), xylo-LNA, a-L-LNA, β-D-LNA, cEt (2'-O,4'-C constrained ethyl) LNA, cMOEt (2'-O,4'-C constrained methoxyethyl) LNA, ethylene-bridged nucleic acid (ENA), tricycio DNA; unlocked nucleic acid (UNA); cyclohexenyl nucleic acid (CeNA), altriol nucleic acid (ANA), hexitol nucleic acid (HNA), fluorinated HNA (F-HNA), pyranosyl-RNA (p-RNA), 3'-deoxypyranosyl-DNA (p-DNA); morpholino (as e.g. in PMO, PPMO, PMOPlus, PMO-X); and their derivatives and any combination thereof.

Possible other modifications may be selected from the group consisting of peptide-base nucleic acid (PNA), boron modified PNA, pyrrolidine-based oxy-peptide nucleic acid (POPNA), glycol- or glycerol-based nucleic acid (GNA), threose-based nucleic acid (TNA), acyclic threoninol-based nucleic acid (aTNA), oligonucleotides with integrated bases and backbones, pyrrolidine-amide oligonucleotides (POMs), linker (e.g. propane, hexane or polyethyleneglycole), brancher (e.g. symmetrical or asymmetrical brancher), lipid (e.g. palmitate, stearate), peptide or dye-modified oligonucleotides as well as Cholesterol- and GalNAc-modified oligonucleotides; and their derivatives and any combination thereof.

In a further set of preferred embodiments herein, the molecules of interest are polypeptides.

In line with the above disclosure, generally, also said polypeptides may or may not be modified herein. Consequently, further embodiments to the particular embodiments for polypeptides described below are envisaged herein, in which respective polypeptides are modified.

Generally, polypeptides are very well known to the person skilled in the art as chains of amino acids linked by peptide bonds. In context with the present invention, polypeptides are defined to have a length of at least ten residues, preferably of at least twenty residues (and in certain embodiments of at least fifty residues). In the present invention, "polypeptides" preferably include "proteins".

Generally herein, polypeptides are not at all limited as the present methods are considered to generally function with various kinds of polypeptides. Preferably, however, the polypeptides are polypeptides that are soluble in water or organic mixtures thereof.

In context with general embodiments of the present invention, polypeptides are proteins, which are preferably selected from the group consisting of an enzyme, a cell receptor ligand, a protein ligand, a signal transduction protein, a cytokine, an antibody, an antibody fragment, an antibody domain, a diabody, an scFv fragment, an sc(Fv)2 fragment, and a chimeric antibody.

All of those polypeptides are well known by the person skilled in the art and their meaning in context with the present invention corresponds to their respective general meaning in the art.

Consequently, in certain embodiments, the molecule of interest is a protein.

In particular embodiments, the molecule of interest is an enzyme.

In other embodiments, the molecule of interest is a cell receptor ligand.

In other embodiments, the molecule of interest is a protein ligand.

In other embodiments, the molecule of interest is a signal transduction protein.

In other embodiments, the molecule of interest is a cytokine.

In further embodiments, the molecule of interest is an antibody.

In other related embodiments, the molecule of interest is an antibody fragment.

In particular embodiments, the molecule of interest is an antibody domain.

In particular embodiments, the molecule of interest is an scFv fragment.

In particular embodiments, the molecule of interest is an sc(Fv)2 fragment.

In even further embodiments, the molecule of interest is a diabody (commonly known as combination of two scFvs connected with short linker peptides).

In even further embodiments, the molecule of interest is a chimeric antibody.

Consequently, in certain non-limiting embodiments herein, the polypeptides comprises from 90 to 1800 amino acid residues, such as from 110 to 1800 amino acids, particularly from 135 to 1800 amino acid residues, especially from 135 to 1350 amino acid.

Similarly, in some non-limiting embodiments herein, the polypeptides have a molecular weight of from 10 to 200 kDa, particularly from 15 to 200 kDa, especially from 15 to 150 kDa.

Also in case of polypeptides, computational methods for calculating the molecular weight of a given oligonucleotide having a given sequence are readily available (e.g. online) to the person skilled in the art.

In general embodiments of the present invention, the polypeptides may be a mixture of polypeptides, such as a mixture of any of the individual types of polypeptides defined herein above. Alternatively the polypeptides may be a mixture of polypeptides of the same type of polypeptide defined herein, such as a mixture of various scFv fragments.

In particular embodiments herein, said molecule of interest is a polypeptide comprising one or more modifications selected from the group consisting of phosphorylation, disulfide bonds, glycosylation, acetylation, amidation, gamma-carboxyglutamic acid hydroxylation, methylation, sulfatation, lipids (particularly selected from myristate, palmitate, farnesyl, geranyl-geranyl, a GPI anchor, N-acyl diglyceride), linkers, dyes, and signal sequences. Preferably, said modification is selected from sugars, lipid, linkers and dyes.

Generally herein, the ultrafiltration membrane used in ultrafiltration is not particularly limited - neither by type nor by size, nor by cut-off value, and suchlike, and may principally freely be selected by the skilled person based on the desired particular application.

In preferred embodiments herein, however, the ultrafiltration membrane has a Molecular Weight Cut-Off (which is also referred to herein as "MWCO") in the range between 1 and 100 kDa, preferably between 1 and 30 kDa, particularly between 1 and 10 kDa. In further particular embodiments, depending on the particular applications as will be appreciated by the skilled artisan, the ultrafiltration membrane has a Molecular Weight Cut-Off in the range between 1 and 7 kDa, particularly between 1 and 5 kDa, especially between 1 and 3 kDa, in particular of about 2 kDa.

In the present invention, generally, a difference in osmolarity on the retentate side and on the permeate side serves as a driving force to increase flux through the ultrafiltration membrane.

Consequently, without intending to be bound by theory, there are principally at least two driving forces leading to a flux through the ultrafiltration membrane, namely the above driving force caused by the osmolarity provided by said osmolytes, as well as the pressure(s) applied during ultrafiltration (preferably TFF or CFF, respectively) itself.

In preferred embodiments, the osmolarity of the solution on the permeate side of the ultrafiltration membrane is at least by a factor of 1.25, particularly at least by a factor of 1.5, especially at least by a factor of 2.0, in particular at least by a factor of 3.0, or at least by a factor of 5.0, or at least by a factor of 10.0, or at least by a factor of 20.0 higher than that of the solution on the retentate side of the ultrafiltration membrane.

Consequently, where the methods of the invention are defined by referring to a step of contacting the permeate side of the ultrafiltration membrane with a solution comprising osmolytes - the osmolarity of the solution on the permeate side of the ultrafiltration membrane is at least by a factor of 1.25, particularly at least by a factor of 1.5, especially at least by a factor of 2.0, in particular at least by a factor of 3.0 higher than that of the solution on the retentate side of the ultrafiltration membrane.

Likewise, where the methods of the invention are defined as comprising providing osmolytes on the permeate side, the osmolarity of the resulting solution on the permeate side is preferably at least by a factor of 1.25, particularly at least by a factor of 1.5, especially at least by a factor of 2.0, in particular at least by a factor of 3.0, or at least by a factor of 5.0, or at least by a factor of 10.0, or at least by a factor of 20.0 higher higher than that of the solution on the retentate side of the ultrafiltration membrane.

Hence, generally, in particular embodiments, said osmolarity on the permeate side is at least by a factor of 1.25 higher than that on the retentate side. In further such embodiments, said osmolarity on the permeate side is at least by a factor of 1.5 higher than that on the retentate side. In further preferred such embodiments, said osmolarity on the permeate side is at least by a factor of 2.0 higher than that on the retentate side, such as at least by a factor of 2.5 higher than that on the retentate side and even at least by a factor of 3.0, or at least by a factor of 5.0, or at least by a factor of 10.0, or at least by a factor of 20.0 higher than that on the retentate side.

As the skilled artisan will appreciate, the osmolarity of the solution on the permeate side of the ultrafiltration membrane changes throughout the methods of the present invention due to the permeate that is passing through the ultrafiltration membrane and by that diluting the osmolyte.

However, in particular embodiments herein, the osmolarity of the solution on the permeate side of the ultrafiltration membrane remains at least by said factor(s) specified herein above throughout the whole method.

In other embodiments, the method is carried out until the osmolarity of the solution on the permeate side of the ultrafiltration membrane corresponds to the one on the retentate side of the ultrafiltration membrane.

In more preferred embodiments, the method is carried out until a certain concentration has been reached. Said certain concentration may also be designated as a desired concentration. It may depend on the particular aims of the given method and may e.g. be tested by taking samples and measuring OD values or suchlike.

Likewise, in particular embodiments herein, the osmolarity of the solution on the permeate side of the ultrafiltration membrane does not drop below said factor(s) indicated herein above throughout the whole method.

In the method of the present invention, said osmolytes may be defined as being provided in a starting solution comprising said osmolytes.

Consequently, in respective embodiments, the osmolarity of said starting solution may be defined as being at least by a factor of 1.5, particularly at least by a factor of 1.75, especially at least by a factor of 2.0, in particular at least by a factor of 3.0, or at least by a factor of 5.0, or at least by a factor of 10.0, or at least by a factor of 20.0, or at least by a factor of 30.0 higher than that of the starting solution comprising said molecules of interest which is employed in the method.

Hence, in particular embodiments said osmolarity of the starting solution comprising said osmolytes is at least by a factor of 1.5 higher than that of the starting solution comprising said molecules of interest which is employed in the method, such as at least by a factor of 1.75 higher than that of the starting solution comprising said molecules of interest which is employed in the method. Particularly, said osmolarity of the starting solution comprising said osmotically active molecules is at least by a factor of 2.0 higher than that of the starting solution comprising said molecules of interest which is employed in the method, such as at least by a factor of 2.5 higher than that of the starting solution comprising said molecules of interest which is employed in the method. More particularly, said osmolarity of the starting solution comprising said osmolytes is at least by a factor of 3.0, or at least by a factor of 5.0, or at least by a factor of 10.0, or at least by a factor of 20.0, or at least by a factor of 30.0 higher than that of the solution comprising said molecules of interest which is employed in the method.

Generally, in one set of embodiments herein, the osmolarity on the retentate side is calculated by taking into account all osmolytes comprised in the respective solution, wherein the osmolarity on the permeate side is calculated by only taking into account said osmolytes having a molecular weight that is higher than the MWCO of the ultrafiltration membrane.

In another set of preferred embodiments herein, each osmolarity (i.e. both that on the retentate side and that on the permeate side) is calculated by only taking into account osmolytes, which have a molecular weight that is higher than the MWCO of the ultrafiltration membrane, comprised in the respective solutions.

In any case, in certain preferred embodiments, said osmolytes having a molecular weight that is higher than the MWCO of the ultrafiltration membrane are provided in a starting solution having a concentration of at least 12,5 mM, in particular of at least 25 mM, particularly of at least 40 mM, especially of at least 50 mM.

That is, in particular embodiments, said osmolytes having a molecular weight that is higher than the MWCO of the ultrafiltration membrane are provided in a starting solution having a concentration of at least 25 mM. In particular embodiments, said osmolytes having a molecular weight that is higher than the MWCO of the ultrafiltration membrane are provided in a starting solution having a concentration of at least 40 mM. In particular embodiments, said osmolytes having a molecular weight that is higher than the MWCO of the ultrafiltration membrane are provided in a starting solution having a concentration of at least 50 mM.

Alternatively, in particular preferred embodiments of the invention, the difference in osmolarity is defined by reference to the molarity of the respective starting solutions on the two sides of the membranes. That is, in particular embodiments, said osmolytes having a molecular weight that is higher than the MWCO of the ultrafiltration membrane are provided in a starting solution, the molarity of which is at least 2 times the molarity of the starting solution comprising said molecules of interest. In other preferred embodiments, said osmolytes having a molecular weight that is higher than the MWCO of the ultrafiltration membrane are provided in a starting solution, the molarity of which is at least 5 times the molarity of the starting solution comprising said molecules of interest.

In the methods of the invention as defined herein above, the methods may comprise one or more preceding steps for ultrafiltrating said molecules of interest, in particular for concentrating said molecules of interest. Said one or more further steps may not involve the use of osmolytes having a molecular weight that is higher than the MWCO of the ultrafiltration membrane on the permeate side of the ultrafiltration membrane. In other words, (regular) ultrafiltration step(s) may be used prior to the method of the invention, i.e. before making use of said osmolytes. In even other words, standard ultrafiltration may be performed with a given solution comprising molecules of interest before the resulting solution comprising molecules of interest is employed as a starting solution comprising molecules of interest in methods of the present invention.

Consequently, in particular embodiments, said step of contacting the permeate side of the ultrafiltration membrane with a solution comprising osmolytes is preceded by one or more further steps for ultrafiltrating said molecules of interest, in particular for concentrating said molecules of interest. Preferably, said one or more further steps comprise TMP-assisted concentration. Said one or more further steps may not involve the use of osmolytes having a molecular weight that is higher than the MWCO of the ultrafiltration membrane on the permeate side of the ultrafiltration membrane. Likewise, in respective embodiments of those methods of the invention, which are defined by a provision of osmolytes, said provision is preceded by one or more steps for ultrafiltrating said molecules of interest, in particular for concentrating said molecules of interest. Said one or more steps may not involve the use of osmolytes having a molecular weight that is higher than the MWCO of the ultrafiltration membrane on the permeate side of the ultrafiltration membrane. Thus, in any case, the methods of the invention may be characterized in that the starting solution comprising said molecules of interest which is employed in the method has previously been subjected to a concentration step (e.g. selected from a precipitation step and an ultrafiltration method), and preferably has previously been subjected to an ultrafiltration method that does not include using said osmolytes, which have a molecular weight that is higher than the MWCO of the ultrafiltration membrane, on the permeate side of the ultrafiltration membrane.

In the above embodiments, generally, said one or more further steps may optionally be carried out in another ultrafiltration device (preferably a TFF or CFF device, respectively) or in the same ultrafiltration device as the obligatory step(s) of the methods of the invention. In other words, said one or more preceding steps may be performed at the same ultrafiltration device as the other steps defining the respective method. Alternatively, any such one or more preceding steps may be performed at another ultrafiltration device before turning for the ultrafiltration device used for said other steps defining the respective method.

In a further set of general embodiments herein, the methods of the invention do not include steps, that do not involve providing said osmolytes, which have a molecular weight that is higher than the MWCO of the ultrafiltration membrane, on the permeate side of the ultrafiltration membrane. In a further set of general embodiments herein, the methods of the invention do not include ultrafiltration steps, that do not involve providing said osmolytes, which have a molecular weight that is higher than the MWCO of the ultrafiltration membrane, on the permeate side of the ultrafiltration membrane.

Alternatively, any such prior steps of ultrafiltration, in particular those described herein above, may also be comprised as integral parts of the methods of the invention.

Moreover, as the person skilled in the art will appreciate, the step of contacting the permeate side of the ultrafiltration membrane with a solution comprising osmolytes in the methods of the present invention may be repeated at least once. In other words, the respective methods of the invention may include multiple such steps. Likewise, in respective embodiments of those methods of the invention, which are defined by a provision of osmolytes, said provision of osmolytes may be repeated at least once. In other words, the respective methods of the invention may include multiple such steps. Thus, in any case, the methods of the invention as defined herein may generally be repeated to further increase the outcome. In particular embodiments, each repetition involves using a new starting solution comprising osmolytes having a molecular weight that is higher than the MWCO of the ultrafiltration membrane.

According to further general embodiments of the methods of the invention, one or more of any method step(s) may be repeated.

Generally herein, the method may involve the use of i) flow-through on the retentate side of the membrane, wherein the solution on the retentate side is not recirculated on the retentate side of the membrane, or (preferably) ii) recirculation on the retentate side of the membrane, wherein the solution on the retentate side is recirculated on the retentate side of the membrane.

Likewise, the method may involve the use of i) flow-through on the permeate side of the membrane, wherein the solution on the permeate side is not recirculated on the permeate side of the membrane, or (preferably) ii) recirculation on the permeate side of the membrane, wherein the solution on the permeate side is recirculated on the permeate side of the membrane.

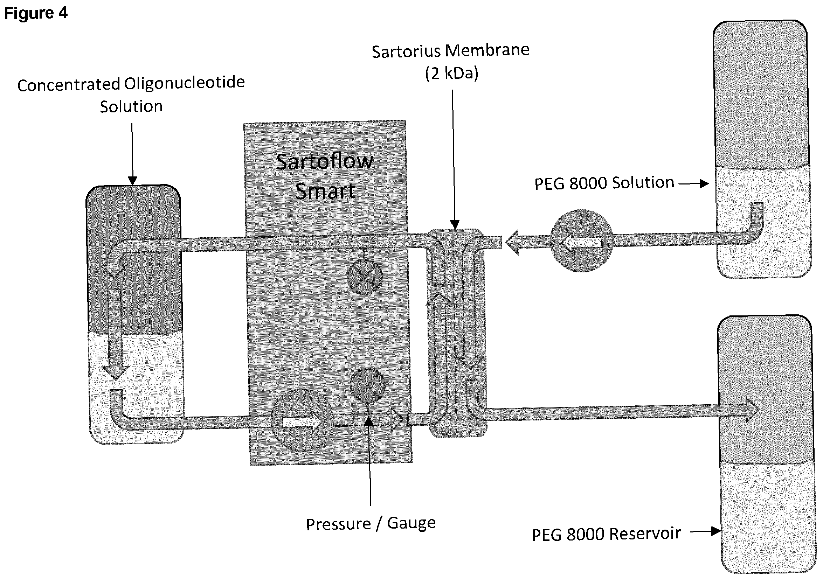

Accordingly, in certain general embodiments herein, the solution comprising said osmolytes is employed by means of a flow-through method, where said solution passes from one reservoir to another. An exemplary specific embodiment to this end is depicted in Figure 4. In certain embodiments, the flow-through occurs gravimetrically. Alternatively, said solution may e.g. be pumped from said one reservoir to another. Consequently, one or more pumps may be used in embodiments not involving circulation (i.e. where the osmolyte is only pumped alongside the ultrafiltration membrane, but without recirculation). Alternatively, the latter procedure may also be attended to without using pump(s), etc. - as will readily be appreciated by the skilled reader.

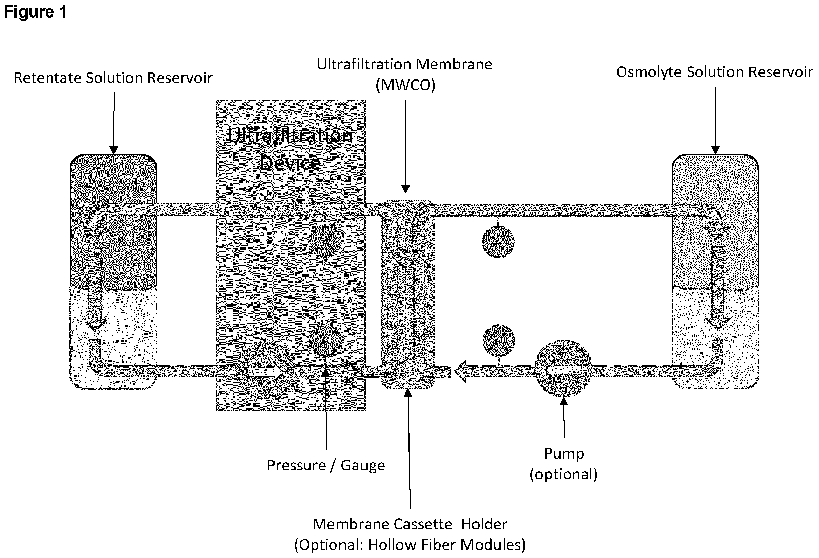

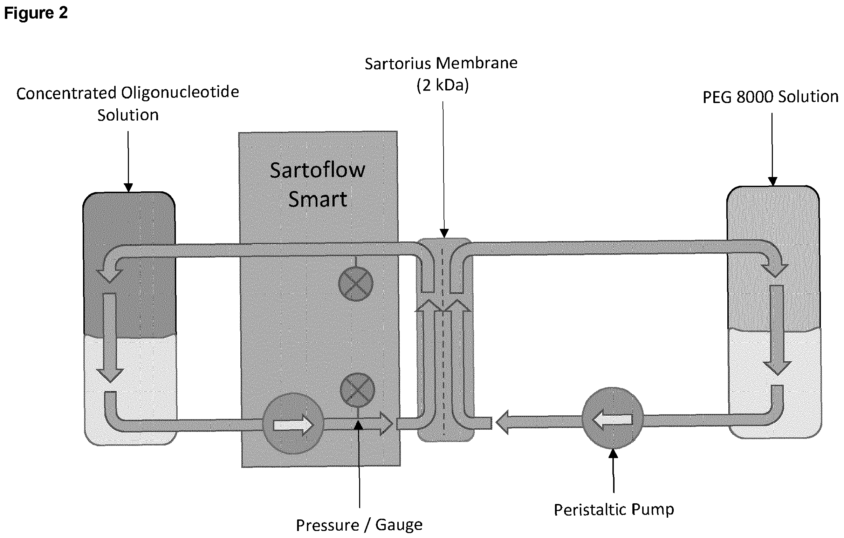

Besides, as the person skilled in the art will appreciate, a circulating of the solution comprising said osmolytes on the permeate side of the membrane may advantageously be used in the present invention. Accordingly, in further preferred general embodiments of the present invention, the methods of the invention comprise circulating the solution comprising said osmolytes on the permeate side of the membrane. Exemplary (specific) embodiments to this end are depicted in Figures 1 and 2.

In particular embodiments, said circulating involves use of a pump and optionally also the use of a reservoir that is part of the circulation and that initially includes the starting solution comprising said osmolytes.

In particular embodiments, the method of the invention comprises the use of one or more pumps. In a particular embodiment, the method of the invention comprises using a pump (which may e.g. be a feed pump, and which is a recirculation pump in certain embodiments) on the retentate side of the membrane, as well as a pump (which is a recirculation pump in certain embodiments) on the permeate side of the membrane. In certain preferred embodiments, the pump(s) is/are (a) peristaltic pump(s).

In any case, in some embodiments, the methods of the invention are performed by employing an ultrafiltration device using circulation on both sides of the membrane for a given time, and optionally until an equilibrium is reached, in which the concentration of the molecules of interest no longer increases.

In preferred embodiments, the methods of the invention are performed until a certain concentration (or desired concentration, respectively) has been reached. Said concentration may depend on the particular aims of the given method and may e.g. be monitored by taking and measuring samples.

Further embodiments of the invention result from replacing the phrase "contacting the permeate side of the ultrafiltration membrane with a solution" in any of the embodiments described herein by the phrase "combining the permeate with a solution", whereas respective particular embodiments correspond to those described above.

As the skilled artisan will appreciate, the total recovery of molecules of interest from ultrafiltration may be improved by rinsing the retentate side of the ultrafiltration membrane and combining the resulting solution with the retentate. Even though such procedure may reduce the final concentration of the molecules of interest, the skilled artisan will readily employ or not employ such measures depending on the circumstances and particular goals to be achieved. Accordingly, in further embodiments of the methods of the invention, the method further comprises a step of rinsing the retentate side of the ultrafiltration membrane to increase the yield of the molecules of interest.

Further with respect to the osmolytes, said osmolytes are preferably polymers. Non-limiting preferred examples of such osmolytes include polyethylene glycols (PEG), polyacrylic acid sodium salt, polyvinyl pyrrolidone, carboxymethyl cellulose, polyvinyl alcohol and mixtures thereof.

In particularly preferred embodiments thereof, the osmolyte is polyethylene glycol (PEG). Without intending to be bound by theory, PEG is particularly useful to this end due to its (high) solubility in water despite its (high) molecular weight. In some particular embodiments, the PEG is mPEG.

In certain preferred embodiments, the osmolyte (such as the PEG) has an average molecular weight of between 1000 and 10,000,000 g/mol, particularly between 1000 and 2,000,000 g/mol, more particularly of between 2000 and 200,000 g/mol, especially of between 2000 and 60,000 g/mol, in particular of between 2000 and 20,000 g/mol.

In some particular non limiting embodiments, the osmolyte (such as the PEG) has an average molecular weight of between 6000 and 12500 g/mol. In more particular embodiments, the osmolyte (such as the PEG) has an average molecular weight of between 7000 and 9000 g/mol. In particular embodiments herein, said PEG has an average molecular weight of about 8000 g/mol. In a given osmolyte (such as PEG) preparation having a given average molecular weight, the exact composition regularly involves osmolyte molecules of various sizes resulting in said average size.

In general embodiments herein, as already depicted above, the osmolyte (such as the PEG) has a molecular weight that exceeds the MWCO of the ultrafiltration membrane. In certain other preferred embodiments herein, the osmolyte (such as the PEG) has an average molecular weight that exceeds the MWCO of the ultrafiltration membrane.

In particular preferred embodiments of both of the above, however, the osmolytes essentially do not pass through the ultrafiltration membrane, which is employed in the methods herein.

As used herein, the phrase according to which molecules (such as the molecules of interest in context with the invention and/or osmolytes in context with the invention) "pass through the membrane" (and suchlike terms) essentially refers to molecules that cross the ultrafiltration membrane due to their size. Generally, molecules which are smaller than the size of the pores of the membrane pass through the membrane. Likewise, generally, molecules which are bigger than the size of the pores of the membrane do not pass through the membrane.

In other words, it may be said that molecules which have a molecular weight that is smaller than the MWCO of the ultrafiltration membrane pass through the membrane. Likewise, it may be said that molecules which have a molecular weight that is higher than the MWCO of the ultrafiltration membrane do not pass through the membrane.

In any case, in preferred embodiments herein, the molecular weights or average molecular weights of the osmolytes, respectively (in combination with the MWCO of a given ultrafiltration membrane) are selected such that the osmolytes essentially do not pass through the ultrafiltration membrane. In certain preferred embodiments herein, less than 10% of the osmolytes, preferably less than 5% of the osmolytes, in particular less than 2% of the osmolytes, especially less than 1% of the, more particularly less than 0.1%, more particularly less than 0.01%, such as less than 0.001% of the osmolytes pass through the ultrafiltration membrane.

Likewise, in certain preferred embodiments herein, the molecules of interest essentially do not pass through the ultrafiltration membrane - even though the latter is oftentimes of less importance for a given application than the former. Consequently, in particular embodiments, less than 20% of the molecules of interest, preferably less than 10% of the molecules of interest, in particular less than 5% of the molecules of interest, especially less than 2% of the molecules of interest, more particularly less than 1% of the molecules of interest, more particularly less than 0.1% of the molecules of interest, more particularly less than 0.01% of the molecules of interest pass through the ultrafiltration membrane.

In any case, the skilled artisan may readily test potential passing through the membrane of molecules by detecting same e.g. by the use of antibodies to ensure that the respective desirable goals are achieved in a given method. Besides, the skilled artisan will readily judge and adjust the degree of retaining the respective molecules based on the particular application pursued with the methods and uses of the invention

As is also apparent from the present Examples, but without intending to be bound by theory, higher concentrations of PEG are considered to improve the outcome of the methods of the invention. Consequently, in preferred embodiments herein, said osmolytes, such as said PEG molecules, are present at a concentration of at least 20 mM, particularly of at least 25 mM, especially of at least 40 mM, especially of at least 50 mM.

Generally, as the methods of the invention are considered to be suitable for various purposes and are considered to be connected with various advantages, the methods of the invention may be characterized as any of the following items. Importantly, as those items are generally not exclusive, particular embodiments of the invention which are preferred herein may be characterized by combinations of any number of the following items. Any such combinations are, thus, clearly considered to also be within the embodiments of the present invention, even though not any combination is literally depicted below. Consequently, any and all of the methods of the invention may be

- a method for concentrating a compound for research purposes; and/or

- a method for concentrating a commercial compound; and/or

- a method for concentrating an adjuvant or excipient; and/or

- a method for concentrating an active pharmaceutical ingredient (API) for incorporation into a medicament; and/or

- a method that does not comprise lyophilization; and/or

- a method that is for avoiding lyophilization; and/or

- a method for improving the yield of molecules of interest (optionally for increasing efficiency of a further step of lyophilization); and/or

- a method for enhancing concentrating said solution comprising said molecules of interest; and/or

- a method for improving the efficiency of the ultrafiltration; and/or

- a method for enhancing osmotic flow through the ultrafiltration membrane; and/or

- a method for drawing solvent molecules (particularly selected from water, organic solvents and mixtures thereof) from the solution comprising said molecule of interest; and/or

- a method for achieving a higher final concentration of the molecule of interest; and/or

- a method for preparing an active pharmaceutical ingredient; and/or

- a method for preparing a medicament; and/or

- an ultrafiltration/diafiltration ("UF/DF") method; and/or

- a method that allows concentrating said solution comprising the molecule of interest to a concentration of at least 150 mg/ml; particularly of at least 200 mg/ml; especially of at least 300 mg/ml; and/or

- a method for enhancing flux through the ultrafiltration membrane.

In particular embodiments, the method of the invention is a method for preparing an active pharmaceutical ingredient (API) for incorporation into a medicament.

In particular embodiments, the method of the invention is a method for concentrating an active pharmaceutical ingredient (API) for incorporation into a medicament.

In particular embodiments, the method of the invention is a method that does not comprise lyophilization.

In particular embodiments, the method of the invention is a method that is suitable for avoiding lyophilization.

In other particular embodiments, however, the method of the invention is a method that is suitable for improving the outcome of a subsequent lyophilization. That is, when obtaining a higher concentrated sample of a molecule of interest using a method of the present invention, and lyophilization is intended for the manufacture of the final product, such a higher concentration before lyophilization advantageously allows obtaining a much higher amount of final product with the same type of lyophilization. As one particular example, if a given product can be produced in a concentration that is three times as high than without the methods of the invention, a subsequent lyophilization step allows obtaining three times the amount of final product as compared to a method not using said methods of the invention. As the skilled reader will appreciate, the lyophilization itself still requires the same use of energy despite the much higher efficiency or yield, respectively.

Accordingly, a further advantage of the methods and uses of the invention is that a subsequent lyophilization may significantly be improved without substantial negative influence on the energy required for lyophilization.

In particular embodiments, the method of the invention is a method for enhancing concentrating said solution comprising said molecules of interest.

In particular embodiments, the method of the invention is a method for improving the efficiency of the ultrafiltration.

In particular embodiments, the method of the invention is a method for enhancing osmotic flow through the ultrafiltration membrane. In particular preferred embodiments herein, the method of the invention is a method for enhancing flux through the ultrafiltration membrane.

In particular embodiments, the method of the invention is a method for drawing solvent molecules (particularly selected from water, organic solvents and mixtures thereof) from the solution comprising said molecule of interest.

As the skilled person will appreciate, also other small molecules such as salts will be drawn from the solution comprising said molecule of interest. This may be desirable or - in certain cases - might not be desirable. In any case, should there be an interest to maintain certain small molecules, the skilled person will be readily in a position to supplement such molecules - e.g. by the use of respective solutions.

In particular embodiments, the method of the invention is a method for achieving a higher final concentration of the molecule of interest. In preferred embodiments, the method of the invention is a method for achieving a higher final concentration of the molecule of interest than achievable with an equivalent method not employing said osmolytes.

In particular embodiments, the method of the invention is a method for preparing an active pharmaceutical ingredient.

In particular embodiments, the method of the invention is a method for preparing a medicament.

In particular embodiments, the method of the invention is an ultrafiltration/diafiltration ("UF/DF") method.

In particular embodiments, the method of the invention is a method that allows concentrating said solution comprising the molecule of interest to a concentration of at least 150 mg/ml; particularly of at least 200 mg/ml; especially of at least 300 mg/ml.

In particular embodiments, the method of the invention is a method for avoiding lyophilization.

In particular embodiments, the method of the invention is a method for enhancing flux through the ultrafiltration membrane.

In a further (i.e. seventh) aspect herein there is provided a use of osmolytes in an method for ultrafiltrating a solution comprising molecules of interest, the method comprising a step of contacting the permeate side of the ultrafiltration membrane with a solution comprising said osmolytes, wherein said osmolytes have a molecular weight that is higher than the MWCO of the ultrafiltration membrane, and wherein the solution on the permeate side of the ultrafiltration membrane has a higher osmolarity than the solution on the retentate side of the ultrafiltration membrane.

In preferred embodiments of said use, the corresponding method for ultrafiltrating is further characterized as defined herein above.

In an even further (i.e. eighth) aspect herein there is provided a use of osmolytes for improving a method for ultrafiltrating a solution comprising molecules of interest, wherein said osmolytes have a molecular weight that is higher than the MWCO of the ultrafiltration membrane, and wherein said osmolytes are provided on the permeate side of the ultrafiltration membrane in order to improve ultrafiltration.

In preferred embodiments of said use, the corresponding method for ultrafiltrating is further characterized as defined herein above.

In addition, in preferred embodiments of any of the uses of the invention as described herein above, said method for ultrafiltrating is a method for concentrating a solution comprising molecules of interest via ultrafiltration.

Generally, as with the methods of the invention outlined above, also the uses according to the invention are considered to be suitable for various purposes and are considered to be connected with various advantages. Consequently, as in case of the methods defined above, any and all of the uses of the invention may for example be for

- enhancing concentrating any solution comprising molecules of interest; and/or for

- improving the efficiency of the ultrafiltration; and/or for

- enhancing osmotic flow through the ultrafiltration membrane; and/or for

- drawing solvent molecules (particularly selected from water, organic solvents or mixtures thereof) from the solution comprising said molecule of interest; and/or for

- achieving a higher final concentration of the molecule of interest; and/or for

- preparing an active pharmaceutical ingredient; and/or for

- preparing a medicament; and/or for

- ultrafiltration/diafiltration of the molecule of interest; and/or for

- concentrating said solution comprising the molecule of interest to a concentration of at least 150 mg/ml, particularly of at least 200 mg/ml, especially of at least 300 mg/ml; and/or for

- avoiding lyophilization; and/or for

- for improving the yield of molecules of interest (optionally for improving the efficiency of a subsequent lyophilization);

- enhancing flux through the ultrafiltration membrane.

Consequently, in particular embodiments the use is for enhancing concentrating said solution comprising said molecules of interest.

In particular embodiments the use is for improving the efficiency of the ultrafiltration.

In particular embodiments the use is for enhancing osmotic flow through the ultrafiltration membrane.

In particular embodiments the use is for drawing solvent molecules (particularly selected from water, organic solvents and mixtures thereof) from the solution comprising said molecule of interest.

In particular embodiments the use is for achieving a higher final concentration of the molecule of interest.

In particular embodiments the use is for preparing an active pharmaceutical ingredient.

In particular embodiments the use is for preparing a medicament.

In particular embodiments the use is for ultrafiltration/diafiltration of the molecule of interest.

In particular embodiments the use allows concentrating said solution comprising the molecule of interest to a concentration of at least 150 mg/ml; particularly of at least 200 mg/ml; especially of at least 300 mg/ml.

In particular embodiments the use is for avoiding lyophilization.

In particular embodiments the use is for enhancing flux through the ultrafiltration membrane.

In a further (i.e. ninth) aspect of the present invention, there is provided an ultrafiltration device for filtrating, particularly for concentrating, a solution comprising molecules of interest, characterized in that the device comprises i) means for contacting the permeate side of the ultrafiltration membrane with a solution comprising osmolytes having a molecular weight that is higher than the MWCO of the ultrafiltration membrane, to increase flux through the ultrafiltration membrane; and/or ii) means for combining the ultrafiltration permeate with a solution comprising osmolytes having a molecular weight that is higher than the MWCO of the ultrafiltration membrane, and (optionally) for circulating the resulting solution on the permeate side of the membrane.

Consequently, in certain embodiments of the ninth aspect, there is provided an ultrafiltration device for filtrating, particularly for concentrating, a solution comprising molecules of interest, characterized in that the device comprises means for contacting the permeate side of the ultrafiltration membrane with a solution comprising osmolytes having a molecular weight that is higher than the MWCO of the ultrafiltration membrane, to increase flux through the ultrafiltration membrane; and optionally for circulating the resulting solution on the permeate side of the membrane.

In other embodiments of the ninth aspect, there is provided an ultrafiltration device for filtrating, particularly for concentrating, a solution comprising molecules of interest, characterized in that the device comprises means for combining the ultrafiltration permeate with a solution comprising osmolytes having a molecular weight that is higher than the MWCO of the ultrafiltration membrane, and (optionally) for circulating the resulting solution on the permeate side of the membrane.

Moreover, in respective combined embodiments of the ninth aspect, there is provided an ultrafiltration device for filtrating, particularly for concentrating, a solution comprising molecules of interest, characterized in that the device comprises means for contacting the permeate side of the ultrafiltration membrane with a solution comprising osmolytes having a molecular weight that is higher than the MWCO of the ultrafiltration membrane, to increase flux through the ultrafiltration membrane; and comprises means for combining the ultrafiltration permeate with a solution comprising osmolytes having a molecular weight that is higher than the MWCO of the ultrafiltration membrane, and (optionally) for circulating the resulting solution on the permeate side of the membrane.

In the above embodiments, any of the means may preferably be characterized in that they do not return the permeate to the retentate side of the ultrafiltration membrane.

In further embodiments of the ultrafiltration devices of the invention, said means for circulation comprise a pump, preferably a peristaltic pump, and a reservoir for the starting solution comprising said osmolytes.

Generally herein, the ultrafiltration device may be configured to operate i) in flow-through mode, wherein the retentate is not recirculated on the retentate side of the membrane, or ii) in recirculation mode, wherein the retentate is recirculated on the retentate side of the membrane, and preferably to operate as in item ii).

Generally herein, the ultrafiltration device may be configured to operate i) in flow-through mode, wherein the permeate is not recirculated on the permeate side of the membrane, or ii) in recirculation mode, wherein the permeate is recirculated on the permeate side of the membrane, and preferably to operate as in item ii).

In further embodiments of the ultrafiltration devices of the invention, the device comprises said starting solution comprising said osmolytes.

In even further embodiments, said ultrafiltration device is further defined in accordance with any of the methods of the invention defined above.

In even further embodiments, said starting solution is further defined as in context with any of the methods of the invention defined above.

EXAMPLES

The invention will now further be described by reference to the following example. Importantly, any examples herein are intended for further illustrating the invention, but are by no means intended to limit the invention in any way.

Example 1

Exemplary experimental procedure for PEG8000-assisted concentration of oligonucleotide solutions is described in detail in the following.

1) The following materials and equipment were used

- Lyophilized and modified 23mer oligonucleotide (Mol. Weight: 7631 g/mol, - Calculated extinction coefficient: 245.174 $mM^{-1}cm^{-1}$ )
- Ultrapure water
- Polyethylenglyocol 8000
- Sartoflow Smart CF system
- Sartocon Slice Hydrosart cassette (Filter area: 0.1 $m^2$, MWCO: 2 kD)
- Sartocon Slice 200 Benchtop peristaltic pump (Sartorius)
- UV spectrophotometer
- HDPE bottles

2) Preparation of oligonucleotide solution

23 g of oligonucleotide solution were dissolved in 1081 mL ultrapure water. Via absorbance measurement (1000-fold dilution) the concentration was determined to be 492 OD/mL (15.3 mg/mL). A total amount of 531.228 OD (16.53g, based on extinction coefficient) of oligonucleotide solution was used for the experiment.

3) Preparation of a 40% (w/w) PEG 8000 solution

80g PEG 8000 were combined with 120g water for the preparation of 200 g 40 % (w/w) PEG 8000 solution.

4) Concentration of oligonucleotide solution

1080.6 mL of oligonucleotide solution were pumped into the Sartoflow Smart CF system (opened retentate valve) and concentrated in four steps (a-d) according to the following parameters.

Phase a) First concentration step

A fresh 1L HDPE permeate bottle was prepared and weighed empty (tare weight: 77g). The differential pressure ($\Delta p$ = Feed pressure - retentate pressure) was set to 0.5 bar and the retentate valve closed until a permeate flow was visible. The pumping rate during this phase is adjusted automatically by the system to meet the differential pressure criterion. This step was continued until around 500 mL of retentate solution were remaining.
After phase a) the permeate valve was closed and the permeate bottle weighed. The total weight was 712g corresponding to a total net weight of 634g (634mL) solution. The calculated amount of oligonucleotide retentate solution after this step thereby was 446 mL.

Phase b) Second concentration step

A fresh 250 mL HDPE bottle was weighed empty (tare weight: 27 g) and installed on the permeate side of the membrane. The CF settings were kept the same as in phase a) until about 330 mL of retentate solution were remaining. After phase b) the pump was stopped and the permeate bottle weighed. The total weight was 171 g corresponding to a total net weight of 144 g (144 mL) water. The calculated amount of oligonucleotide retentate solution after this step thereby was 302 mL.

Phase c) Third concentration step (TMP = 3 bar)

A fresh 250 mL HDPE bottle was weighed empty (tare weight: 27 g) and installed on the permeate side of the membrane. To reach a TMP of approximately 3 bar the retentate valve was closed further to increase the TMP. This phase was monitored since no automatic stop of the pump occurs when the TMP exceeds 3bar. This phase was continued until no longer permeate flow was visible at max. 3 bar TMP. The pump was stopped and the permeate bottle weighed.

The total weight was 163 g corresponding to a total net weight of 136 g (136 mL) water. The calculated amount of oligonucleotide solution after this step thereby was 165 mL

Phase d) Fourth concentration step (TMP = 4bar)

A fresh 250 mL HDPE bottle was weighed empty (tare weight: 27 g) and installed on the permeate side of the membrane. The maximal feed pressure is 4 bar at the Sartoflow Smart system. To gradually increase feed pressure the retentate valve was closed further. No further concentration of the sample is possible after the maximal feed pressure is reached. The pump was stopped after reaching 4bar and the permeate bottle was weighed. The total weight was 60 g corresponding to a total net weight of 33 g (33 mL) water. The calculated amount of oligonucleotide solution after this step thereby was 132 mL.

After phase d) the absorbance of all permeate fractions was measured to determine the total oligonucleotide loss due to breakthrough. The OD values were the following:

Permeate fraction 1: 880 OD
Permeate fraction 2: 2.770 OD
Permeate fraction 3: 7.943 OD
Permeate fraction 4: 6.019 OD
Sum of losses during concentration phase: 17.612 OD (~ 3%)
Remaining amount of oligonucleotide in retentate: 513617 OD
Calculated oligonucleotide concentration in retentate: 3879 OD/mL = 121 mg/mL

5) Osmotic pressure assisted concentration of oligonucleotide solution

A 500 mL glass bottle was weighed empty (tare weight: 396 g) and filled with 207 g of the 40 % (w/w) PEG 8000 solution. The PEG solution was connected to the permeate side of the membrane via silicon tubings. Via a peristaltic pump the PEG solution was circulated on the permeate side of the membrane. The retentate valve was opened fully and the oligonucleotide solution pumped without any retentate pressure over the membrane. Through the osmotic pressure a permeate flow was generated draining water out of the oligonucleotide solution. The pumping was continued until no more significant flow through the membrane could be observed (about 2,5 hours). The pump was stopped and the permeate bottle weight. The total weight was 685 g corresponding to a total net gain of 80.9g (80.9 mL) of water on the permeate side. The calculated amount of oligonucleotide solution in the retentate after this step was thereby 52 mL

The absorbance value of the PEG solution was measured to determine the loss of oligonucleotide in the retentate due to breakthrough. The absorbance value was corrected against an equally diluted PEG solution as blank measurement.

PEG solution breakthrough: 12.834 OD
Sum of losses during first concentration phase and PEG-assisted concentration: 30.444 OD (~6%)
Sum of losses during PEG-assisted concentration: 12.832 OD (~3%)
Calculated amount of oligonucleotide in retentate solution: 500.784 OD
Calculated oligonucleotide concentration in retentate: 9.724 OD/mL = 303 mg/mL

Ther overall osmotic pressure assisted concentration of the oligonucleotide solution: 303 [mg/ml] / 121 [mg/ml] × 100 [%] = 250 [%]

LIST OF REFERENCES

(1) Muslehiddinoglu et al., Technical Considerations for Use of Oligonucleotide Solution API, Nucleic Acid Therapeutics 2020, 30, 189-197

**Claims**

1. A method for ultrafiltrating a solution comprising molecules of interest, the method comprising a step of contacting the permeate side of the ultrafiltration membrane with a solution comprising osmolytes having a molecular weight that is higher than the molecular weight cut-off (MWCO) of the ultrafiltration membrane, wherein the solution on the permeate side of the ultrafiltration membrane has a higher osmolarity than the solution on the retentate side of the ultrafiltration membrane.

2. A method for ultrafiltrating a solution comprising molecules of interest, **characterized in that** it comprises providing osmolytes, which have a molecular weight that is higher than the MWCO of the ultrafiltration membrane, on the permeate side of the ultrafiltration membrane, in order to improve ultrafiltration.

3. The method of any one of claims 1 and 2, wherein the method is a method for concentrating a solution comprising molecules of interest via ultrafiltration, in particular wherein ultrafiltration is tangential flow filtration.

4. The method of any one of the preceding claims, wherein the method allows concentrating said molecules of interest to a concentration of at least 100 mg/ml, particularly of at least 150 mg/ml, especially of at least 200 mg/ml, in particular of at least 250 mg/ml, more in particular of at least 300 mg/ml, more in particular of at least 350 mg/ml, especially of at least 400 mg/ml, such as of at least 450 mg/ml.

5. The method of any one of the preceding claims, wherein said molecule of interest is optionally modified,

   particularly wherein said molecule of interest is

   i) an oligonucleotide, which may or may not be modified, and which is preferably selected from the group consisting of DNA, RNA, mixtures thereof (such as heteroduplexes), particularly wherein said oligonucleotide is single stranded or double stranded, especially wherein the RNA is selected from the group consisting of RNAzymes, siRNA, DsiRNA, shRNA, miRNA, anti-miRNA, mRNA, rRNA, tRNA and sgRNA;
   or
   ii) a polypeptide, which may or may not be modified, and which is preferably selected from the group consisting of a an enzyme, a cell receptor ligand, a protein ligand a signal transduction protein, a cytokine, an antibody, an antibody fragment, an antibody domain, a diabody, an scFv fragment, an sc(Fv)2 fragment, and a chimeric antibody;

   especially wherein said molecule of interest is an active pharmaceutical ingredient (API).

6. The method of any one of the preceding claims, wherein the ultrafiltration membrane has a MWCO in the range between 1 and 100 kDa, particularly between 1 and 30 kDa, especially between 1 and 10 kDa.

7. The method of any one of the preceding claims, wherein the osmolarity of the solution on the permeate side of the ultrafiltration membrane is at least by a factor of 1.25, preferably at least by a factor of 1.5, more preferably of at least by a factor of 2.0, even more preferably of at least by a factor of 3.0 higher than that of the solution on the retentate side of the ultrafiltration membrane.

8. The method of any one of the preceding claims, wherein the osmotically active molecule is selected from the group consisting of polyethylene glycol (PEG), polyacrylic acid (PAA) sodium salt, polyvinyl pyrrolidone, carboxymethyl cellulose, polyvinyl alcohol, and a mixture thereof,
particularly wherein the osmolyte is polyethylene glycol (PEG), particularly PEG having an average molecular weight of between 1000 and 10.000.000 g/mol, particularly between 2000 and 2.000.000 g/mol, particularly of between 2000 and 200.000 g/mol, especially of between 2000 and 60.000 g/mol, in particular of between 2000 and 20.000 g/mol, such as of between 6000 and 12.500 g/mol, of between 7000 and 9000 g/mol, in particular of about 8000 g/mol.

9. The method of any one of the preceding claims, wherein

   i) said method comprises one or more preceding steps for ultrafiltrating said molecules of interest, in particular for concentrating said molecules of interest,
   particularly wherein said one or more preceding further steps do not involve the use of osmolytes having a molecular weight that is higher than the MWCO of the ultrafiltration membrane on the permeate side of the ultrafiltration membrane;
   and/or
   ii) wherein one or more of any method step(s) are repeated.

10. A method for preparing a concentrated solution of molecules of interest from a starting solution of said molecules of interest, wherein the method employs ultrafiltration,

wherein the method comprises a step of contacting the permeate side of the ultrafiltration membrane with a solution comprising osmolytes having a molecular weight that is higher than the MWCO of the ultrafiltration membrane,

wherein the solution on the permeate side of the ultrafiltration membrane has a higher osmolarity than the solution on the retentate side of the ultrafiltration membrane;

particularly wherein the method is further characterized as defined in any of claims 1 to 9.

11. A method for preparing a concentrated solution of molecules of interest from a starting solution of said molecules of interest, wherein the method employs ultrafiltration and wherein the method is **characterized in that** concentrating comprises providing osmolytes on the permeate side of the ultrafiltration membrane which osmolytes have a molecular weight that is higher than the MWCO of the ultrafiltration membrane;

particularly wherein the method is further characterized as defined in any of claims 1 to 9.

12. The method of any one of the preceding claims, wherein said method is

i) a method for enhancing concentrating said solution comprising said molecules of interest;
ii) a method for improving the efficiency of the ultrafiltration;
iii) a method for enhancing osmotic flow through the ultrafiltration membrane;
iv) a method for drawing solvent molecules (particularly selected from water, organic solvents and mixtures thereof) and/or other small molecules (particularly selected from salts) from the solution comprising said molecule of interest;
v) a method for achieving a higher final concentration of the molecule of interest;
vi) a method for preparing an active pharmaceutical ingredient;
vii) a method for preparing a medicament;
viii) an ultrafiltration/diafiltration method;
ix) a method that allows concentrating said solution comprising the molecule of interest to a concentration of at least 150 mg/ml; particularly of at least 200 mg/ml; especially of at least 300 mg/ml;
x) a method for avoiding lyophilization;
and/or
xi) a method for enhancing flux through the ultrafiltration membrane.

13. Use of osmolytes in an method for ultrafiltrating a solution comprising molecules of interest, the method comprising a step of contacting the permeate side of the ultrafiltration membrane with a solution comprising said osmolytes, wherein said osmolytes have a molecular weight that is higher than the MWCO of the ultrafiltration membrane, and wherein the solution on the permeate side of the ultrafiltration membrane has a higher osmolarity than the solution on the retentate side of the ultrafiltration membrane,

particularly wherein the ultrafiltrating is for concentrating said solution comprising molecules of interest, especially wherein said use is for

i) enhancing concentrating said solution comprising molecules of interest;
ii) improving the efficiency of the ultrafiltration;
iii) enhancing osmotic flow through the ultrafiltration membrane;
iv) drawing solvent molecules (particularly selected from water, organic solvents and mixtures thereof) from the solution comprising said molecule of interest;
v) achieving a higher final concentration of the molecule of interest;
vi) for preparing an active pharmaceutical ingredient;
vii) preparing a medicament;
viii) ultrafiltration/diafiltration of the molecule of interest;
ix) concentrating said solution comprising the molecule of interest to a concentration of at least 150 mg/ml, particularly of at least 200 mg/ml, especially of at least 300 mg/ml;
x) avoiding lyophilization;
and/or
xi) enhancing flux through the ultrafiltration membrane,

in particular wherein the use is further characterized as defined in any one of claims 1 to 11.

14. Use of osmolytes in a method for ultrafiltrating a solution comprising molecules of interest, wherein said osmolytes have a molecular weight that is higher than the MWCO of the ultrafiltration membrane, and wherein said osmolytes are provided on the permeate side of the ultrafiltration membrane in order to improve ultrafiltration,

particularly wherein ultrafiltrating is for concentrating said solution comprising molecules of interest, especially wherein said use is for

i) enhancing concentrating said solution comprising molecules of interest;
ii) improving the efficiency of the ultrafiltration;
iii) enhancing osmotic flow through the ultrafiltration membrane;
iv) drawing solvent molecules (particularly selected from water, organic solvents and mixtures thereof) and/or other small molecules (particularly selected from salts) from the solution comprising said molecule of interest;
v) achieving a higher final concentration of the molecule of interest;
vi) for preparing an active pharmaceutical ingredient;
vii) preparing a medicament;
viii) ultrafiltration/diafiltration of the molecule of interest;
ix) concentrating said solution comprising the molecule of interest to a concentration of at least 150 mg/ml, particularly of at least 200 mg/ml, especially of at least 300 mg/ml;
x) avoiding lyophilization;
and/or
xi) enhancing flux through the ultrafiltration membrane,

in particular wherein the use is further characterized as defined in any one of claims 1 to 11.

15. An ultrafiltration device for filtrating, particularly for concentrating, a solution comprising molecules of interest, **characterized in that** the device comprises

i) means for contacting the permeate side of the ultrafiltration membrane with a solution comprising osmolytes having a molecular weight that is higher than the MWCO of the ultrafiltration membrane, to increase flux through the ultrafiltration membrane;
and/or
ii) means for combining the ultrafiltration permeate with a solution comprising osmolytes having a molecular weight that is higher than the MWCO of the ultrafiltration membrane, and (optionally) for circulating the resulting solution on the permeate side of the membrane;
particularly wherein said means are **characterized in that** they do not return the permeate to the retentate side of the ultrafiltration membrane,
especially wherein said means for circulation comprise a pump and a reservoir for the starting solution comprising said osmolytes,
in particular wherein the device comprises said starting solution comprising said osmolytes, wherein said starting solution has a higher osmolarity than the starting solution comprising the molecule of interest to be ultrafiltrated with said device.

**Figure 1**

EP 4 349 459 A1

**Figure 2**

**Figure 3**

Retentate weight during PEG8000-assisted concentration

EP 4 349 459 A1

**Figure 4**

Europäisches
Patentamt

European
Patent Office

Office européen
des brevets

**EUROPEAN SEARCH REPORT**

Application Number

EP 22 19 9848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2015 108501 A1 (SARTORIUS STEDIM BIOTECH GMBH [DE]) 1 December 2016 (2016-12-01) * paragraphs [0004], [0050], [0051], [0054]; examples 1,2 * * paragraphs [0014], [0027] – [0031], [0033], [0034]; figure 2 * | 1-15 | INV. B01D61/00 B01D61/18 B01D69/02 |
| A | EP 3 395 430 A1 (ACCIONA AGUA S A [ES]) 31 October 2018 (2018-10-31) * paragraphs [0017], [0030]; claims 1-3; figure 1 * | 9 | |
| A | KR 2016 0017173 A (KIM JEONG MOON ALOE CO LTD [KR]) 16 February 2016 (2016-02-16) * paragraphs [0040], [0014]; figure 2 * | 9 | |
| A | JP 2016 190228 A (OSAKA GAS CO LTD; MATSUYAMA HIDETO ET AL.) 10 November 2016 (2016-11-10) * paragraph [0044]; figures 1,2 * | 9 | TECHNICAL FIELDS SEARCHED (IPC) |
| A | MING MING LING ET AL: "Novel dual-stage FO system for sustainable protein enrichment using nanoparticles as intermediate draw solutes", JOURNAL OF MEMBRANE SCIENCE, ELSEVIER BV, NL, vol. 372, no. 1, 2 February 2011 (2011-02-02), pages 201-209, XP028370739, ISSN: 0376-7388, DOI: 10.1016/J.MEMSCI.2011.02.003 [retrieved on 2011-02-11] * abstract * * section 2.2 * | 9 | B01D |

-/--

The present search report has been drawn up for all claims

| Place of search | Date of completion of the search | Examiner |
|---|---|---|
| Munich | 28 February 2023 | Hennebrüder, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 22 19 9848**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TWARDOWSKI Z J ET AL:   "POLY ANIONS AND GLUCOSE AS OSMOTIC AGENTS IN SIMULATED PERITONEAL DIALYSIS", ARTIFICIAL ORGANS, BLACKWELL SCIENTIFIC PUBLICATIONS, INC., BOSTON, US, vol. 7, no. 4, 1 January 1983 (1983-01-01) , pages 420-427, XP009081105, ISSN: 0160-564X * abstract; figure 3 * ----- | 1,2, 10-14 | |

TECHNICAL FIELDS
SEARCHED (IPC)

The present search report has been drawn up for all claims

| Place of search | Date of completion of the search | Examiner |
|---|---|---|
| Munich | 28 February 2023 | Hennebrüder, K |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                                                                          
& : member of the same patent family, corresponding
   document

EPO FORM 1503 03.82 (P04C01)

page 2 of 2

EP 4 349 459 A1

## ANNEX TO THE EUROPEAN SEARCH REPORT
## ON EUROPEAN PATENT APPLICATION NO.

EP 22 19 9848

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2023

| Patent document cited in search report | Publication date | Patent family member(s) | Publication date |
|---|---|---|---|
| DE 102015108501 A1 | 01-12-2016 | DE 102015108501 A1 | 01-12-2016 |
| | | WO 2016193100 A1 | 08-12-2016 |
| EP 3395430 A1 | 31-10-2018 | EP 3395430 A1 | 31-10-2018 |
| | | ES 2619113 A1 | 23-06-2017 |
| | | MA 44157 A | 31-10-2018 |
| | | WO 2017109260 A1 | 29-06-2017 |
| KR 20160017173 A | 16-02-2016 | NONE | |
| JP 2016190228 A | 10-11-2016 | JP 6512900 B2 | 15-05-2019 |
| | | JP 2016190228 A | 10-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MUSLEHIDDINOGLU et al.** Technical Considerations for Use of Oligonucleotide Solution API. *Nucleic Acid Therapeutics,* 2020, vol. 30, 189-197